# EUROPÄISCHE PATENTSCHRIFT

(11) **EP 0 863 139 B1**
(45) Veröffentlichungstag und Bekanntmachung des Hinweises auf die Patenterteilung: **28.03.2001**
(21) Anmeldenummer: 98102312.0
(22) Anmeldetag: 11.02.1998
(51) Int. Cl.: C07D 265/26, C07K 5/06, C07D 499/68, A61K 31/535

(54) **Benzoxazindionderivate, Verfahren zu ihrer Herstellung und ihre Verwendung**
Benzoxazindione derivatives, process for their preparation and their use
Dérivés de benzoxazine, leur procédé de préparation et leur application

(30) Priorität: 05.03.1997 DE 19708846
(43) Veröffentlichungstag der Anmeldung: 09.09.1998
(73) Patentinhaber: Grünenthal GmbH, 52078 Aachen (DE)
(72) Erfinder: Heinisch, Lothar, Dr., 07743 Jena (DE); Wittmann, Steffen, Dr., 07743 Jena (DE); Möllmann, Ute, Dr., 07749 Jena (DE); Reissbrodt, Rolf, Dr., 38855 Wernigerode (DE)

(56) Entgegenhaltungen:
- EP-A- 0 341 948
- EP-A- 0 472 062
- EP-A- 0 496 332
- WO-A-97/49670
- DE-B- 1 147 583

## Beschreibung

Die vorliegende Erfindung betrifft neue Benzoxazindionderivate die mit Wirkstoffen, z. B. Antibiotika konjugiert sind. Die Verbindungen sind wirksam als Siderophore bei gramnegativen Bakterien, d.h. sie können Bakterien mit Eisenionen versorgen und als Konjugate mit Wirkstoffen, z. B. mit Antibiotika (als "Siderophor-Antibiotikakonjugate") diese über Eisentransportwege in die Bakterienzelle einschleusen und somit deren Wirksamkeit verbessern bzw. erweitern. Damit soll ein Beitrag geleistet werden zur Bekämpfung penetrationsbezogener Antibiotikaresistenz. Die erfindungsgemäßen Verbindungen stellen Vorstufen für Eisenchelatoren bzw. heterocyclisch geschützte Catecholverbindungen dar, d. h. sie können enzymatisch in entsprechende Catecholderivate umgewandelt werden. Sie können somit den biologischen Eisenstoffwechsel in verschiedener Weise beeinflussen. Anwendungsgebiet der Erfindung ist die pharmazeutische Forschung und Industrie.

Benzoxazindionderivate der Formel **l** mit den angegebenen Substituenten sind bisher in der Literatur nicht beschrieben. Die Verbindungen stellen im Prinzip acylierte Catecholderivate dar, deren eine Acylkomponente in einen Benzoxazinring eingebunden ist.

Als strukturverwandte Verbindungen sind in DE 11 47 583 Salicylsäurederivate beschrieben, die analgetisch wirksam sind. Weiterhin sind aus der Patentliteratur verschiedene Veröffentlichungen bekannt, die verbesserte antibiotische Wirkstoffe zum Ziel haben, z.B. β-Lactam-Verbindungen mit bestimmten Catechol- oder Hydroxypyridonresten aus EP 0 472 062, Cephalosporinverbindungen enthaltend eine Amid- oder Sulfonamidgruppe aus EP 0 341 948 oder Alpha-Aminochinolinoyl-(3)-penicilline aus EP 0 496 332. Eine Siderophorwirksamkeit dieser Verbindungen ist nicht beschrieben.

Es ist bekannt, daß bestimmte Catecholstrukturen in natürlichen Siderophoren als eisenkomplexierende Strukturelemente eine wesentliche Rolle spielen ("Iron Transport in Microbes, Plants and Animals", Hrsg.: Winkelmann, G., van Helm, D., Neilands, J. B., V.Ch.-Verlagsgesellschaft Weinheim, 1987), z. B. ist das Enterobactin, ein Siderophor bei *E.coli* und anderen Bakterienstämmen, ein Trimeres aus N-(2,3-Dihydroxybenzoyl)-L-serin. Auch das Monomer ist als Siderophor wirksam (Hantke, K., FEMS Microbiol. Lett. 67 (1990), 5).

Das N-(2,3-Dihydroxybenzoyl)glycin ist als Siderophor bei *B.subtilis* gefunden worden (lto, T., Neilands, J.B., J. Amer. Chem. Soc. 80 (1958), 4645). Einige catecholsubstituierte Aminosäurederivate sind bereits synthetisch hergestellt worden, z. B. das N-(2,3-Dihydroxybenzoyl)-L-threonin (Kanai, F., Kaneko, T., Morishima, H., Isshiki, K., Takita, T., Takeuchi, T., Umezawa, H., J. Antibiot. 38 (1985), 39), das N²,N⁶-Bis-(2,3-Dihydroxybenzoyl)-L-lysin (Corbin, J.L., Bulen,W.A., Biochemistry 8 (1969), 757; McKee, J.A., Sharma, S.K., Miller, M.J.; Bioconjugate Chem., 2 (1991) 281) und N²,N⁶-Bis-(2,3-dihydroxybenzoyl)-lysyl-N⁶-(2,3-dihydroxy-benzoyl)lysin (Chimiak, A., Neilands, J.B., Structure and Bonding, 58 (1984), 89). Weiterhin wurde bekannt, daß bei verschiedenen Bakterienstämmen bestimmte Glyoxylsäurebenzhydrazone, Oxanilsäurederivate u.a. als Siderophore dienen können (Reissbrodt, R., Heinisch, L., Möllmann, U., Rabsch, W., Ulbricht, H., BioMetals, 6 (1993), 155).

Verschiedene Catecholverbindungen wurden mit ß-Lactamen verknüpft, wodurch eine beträchtliche Steigerung der antibakteriellen Wirksamkeit dieser Antibiotika erzielt wurde, bedingt durch eine Einschleusung über bakterielle Eisentransportwege in die Bakterienzelle (z. B. WO 97/49670 oder Arisawa, M., Sekine, Y., Shimizu, S., Takano, H., Angehrn, P., Then, R.L., Antimicrob. Agents Chemother. 35 (1991), 653). Bisher sind jedoch keine derartigen Verbindungen zu einer klinischen Anwendungsreife gelangt. Zur Erreichung dieses Zieles muß nach weiteren neuen synthetischen Siderophoren gesucht werden, die zur Konjugatbildung mit Antibiotika geeignet sind.

Eine Herstellung von Benzoxazinderivaten aus Acyloxybenzoylchlorid und Aminkomponenten unter Abspaltung von HCI und Methanol ist bisher nicht beschrieben worden.

Die Erfindung dient zur Auffindung von neuen Benzoxazindionderivaten sowie zu ihrer Verwendung. Mit der Erfindung wird angestrebt, geeignete Verbindungen zur Einschleusung von Wirkstoffen, z. B. von Antibiotika über bakterielle Eisentransportwege in die Bakterienzelle zu entwickeln. Durch die Einbindung der Catecholstruktur in die heterocyclische Benzoxazinstruktur soll erreicht werden, daß die Verbindungen in ihrer acylierten Form, insbesondere ihre Konjugate mit Antibiotika, gegenüber den freien Catecholen verbesserte pharmakologische Eigenschaften erhalten bzw. als pharmakologische Transportformen für die eigentlich penetrationsfördernden Catecholverbindungen dienen können.

Der Erfindung liegt die Aufgabe zugrunde, neue Benzoxazindionderivate aufzufinden, die als Siderophore fungieren können.

Die Aufgabe wird erfindungsgemäß gelöst, indem neue Benzoxazindionderivate der allgemeinen Formel **l** bereitgestellt werden, 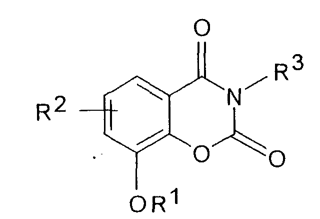 in welcher R¹ = H, COAlkyl oder COOAlkyl, wobei Alkyl jeweils geradkettiges oder verzweigtes C₁₋₈-Alkyl bedeutet, R² = H, geradkettiges oder verzweigtes C₁₋₈-Alkyl, Halogen und R³ folgende Substituenten darstellen:
a) R³ = -Z-CHR⁴-COR⁵
   mit Z = 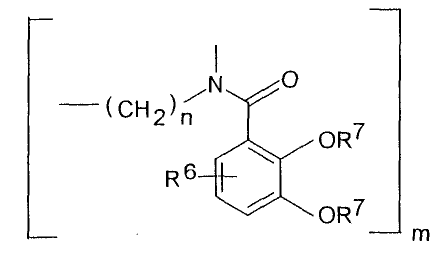 mit R⁴ = H, geradkettiges oder verzweigtes C₁₋₈-Alkyl, Phenyl oder substituiertes Phenyl, das durch geradkettiges oder verzweigtes C₁₋ ₈-Alkyl, Halogen, insbesondere Cl oder F, geradkettiges oder verzweigtes C₁₋₈-Alkoxy, Hydroxy, Carboxy, geradkettiges oder verzweigtes C₁₋₈-Alkoxycarbonyl oder halogensubstituiertes C₁₋₈-Alkyl substituiert ist, insbesondere Hydroxy-oder Acyloxyphenyl, wobei Acyl geradkettiges oder verzweigtes C₁₋₆-Alkanoyl oder geradkettiges oder verzweigtes C₁₋₆-Alkoxycarbonyl bedeutet, oder mit R⁴ = (CH₂)ₙCOX mit X = OA, wobei A = H, geradkettiges oder verzweigtes C₁₋₈-Alkyl, ein Natrium- oder Kaliumion oder ein Ammoniumion bzw. ein ein- bis vierfach alkylsubstituiertes Ammoniumion ist, oder mit X = ein Wirkstoffrest, der den Rest eines Antibiotikums, welches über eine freie OH- oder NH-Gruppe direkt oder über gebräuchliche Linkergruppen mit dem restlichen Strukturteil der Verbindung der Formel **l** verbunden ist, ausgewählt aus der β-Lactam-Antibiotika, wobei diese insbesondere Penicilline, Cephalosporine oder Carbapeneme sind, Tetracycline, Aminoglykoside, Makrolide oder Chinolone umfassenden Gruppe bedeutet, und mit n = 1- 10,
   oder mit R⁴ = (CH₂)ₙ-Y, wobei Y einen Benzoxazindionrest der Form 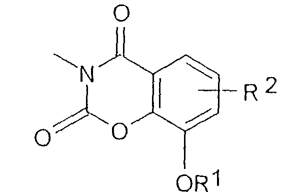 darstellt, wobei R¹ und R² wie oben definiert sind, und beide Benzoxazindionreste gleich oder verschieden sein können, und n = 1- 10 sein kann,
   und mit R⁵ = OA, wobei A wie oben definiert ist oder Benzyl bedeutet,
   oder mit R⁵ = ein Wirkstoffrest wie oben definiert
   oder mit R⁵ = NH-CHR⁸-COR⁹, mit R⁸ = H, geradkettiges oder verzweigtes C₁₋₈-Alkyl, Phenyl oder substituiertes Phenyl wie oben definiert und mit R⁹ = OA, wobei A wie oben definiert ist, oder mit R⁹ = ein Wirkstoffrest wie oben definiert
   oder mit R⁵ = 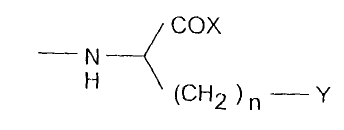 = R¹⁷ mit X und Y wie oben, n = 1 - 10
   und mit R⁶ = H, geradkettiges oder verzweigtes C₁₋₈- Alkyl, Halogen und mit R⁷ = H, Acyl wie oben definiert und
   n = 1 - 10 und m = 1 - 2, mit der Maßgabe, daß wenn R⁴ keinen Wirkstoffrest wie oben definiert enthält, R⁵ oder R⁹ einen Wirkstoffrest wie oben definiert darstellen oder enthalten, oder wenn R⁵ keinen Wirkstoffrest wie oben definiert enthält, R⁴ = (CH₂)ₙCOX mit X = ein Wirkstoffrest wie oben definiert bedeutet, oder
b) R³ = CHR⁴-COR⁵
   mit R⁴ und R⁵ wie oben oder R⁴ = CH₂OH mit der Maßgabe, daß wenn R⁴ keinen Wirkstoffrest wie oben definiert enthält, R⁵ oder R⁹ einen Wirkstoffrest wie oben definiert darstellen, oder wenn R⁵ keinen Wirkstoffrest wie oben definiert enthält, R⁴ = (CH₂)ₙCOX mit X = ein Wirkstoffrest wie oben definiert bedeutet, oder
c) R³ = 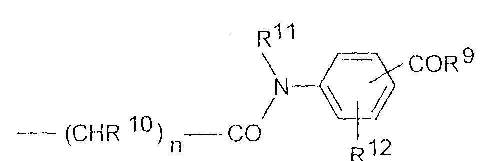 = R¹⁸
   mit R¹⁰ und/oder R¹¹ = H, geradkettiges oder verzweigtes C₁₋₈-Alkyl, Phenyl oder substituiertes Phenyl wie oben definiert, n = 1-10, und mit COR⁹ und R¹² in allen möglichen Positionen, R⁹ = ein Wirkstoffrest wie oben definiert und R¹² = H, geradkettiges oder verzweigtes C₁₋₈-Alkyl, Halogen, Hydroxy, geradkettiges oder verzweigtes C₁₋₈-Alkoxy, ein Benzoxazindionrest Y oder R¹² = 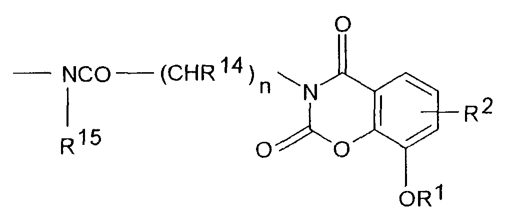 = R¹⁹
   mit R¹, R² wie oben, R¹⁴, R¹⁵ wie R¹, R² und n = 1 - 10 sein kann, oder
d) R³ = 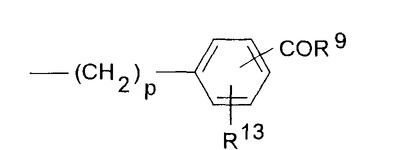 = R²⁰
   mit R¹³ und COR⁹ in allen möglichen Positionen und mit R¹³ = H, geradkettiges oder verzweigtes C₁₋₈-Alkyl, Halogen, Hydroxy, geradkettiges oder verzweigtes C₁₋₈-Alkoxy oder ein Benzoxazindionrest Y, und
   R⁹ = ein Wirkstoffrest wie oben definiert und p= 0 - 2, oder
e) R³ = 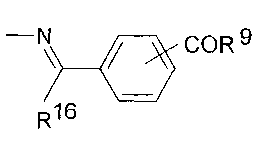 = R²¹ oder R³ = 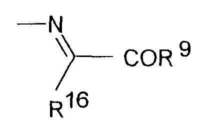 = R²² mit R⁹ = ein Wirkstoffrest, wie oben definiert, R¹⁶ = H, geradkettiges oder verzweigtes C₁₋₈-Alkyl, Phenyl oder substituiertes Phenyl wie oben definiert, oder
f) R³ = ein Wirkstoffrest wie oben definiert

In den vorstehenden Formeln und im folgenden bedeutet Acyl insbesondere geradkettiges oder verzweigtes C₁₋₆-Alkanoyl oder geradkettiges oder verzweigtes C₁₋₆-Alkoxycarbonyl; geradkettiges oder verzweigtes Alkyl und geradkettiges oder verzweigtes Alkoxy, auch in Wortkombinationen wie geradkettiges oder verzweigtes Alkoxycarbonyl, sind insbesondere geradkettiges oder verzweigtes C₁₋₈-Alkyl bzw. -Alkoxy; ein substituiertes Phenyl bedeutet ein durch geradkettiges oder verzweigtes Alkyl, Halogen, insbesondere Cl oder F, geradkettiges oder verzweigtes Alkoxy, Hydroxy, Carboxy, geradkettiges oder verzweigtes Alkoxycarbonyl und halogensubstituiertes Alkyl substituiertes Phenyl. Ein substituiertes Ammoniumion ist ein durch Alkyl ein- oder mehrfach, wie ein- bis vierfach, substituiertes Ammoniumion. Ein Alkalimetallion kann ein Natrium- oder Kaliumion sein.

Ein Wirkstoffrest bedeutet den Rest eines geeigneten antibakteriellen Wirkstoffes mit freier NH- oder OH-Gruppe, wobei der Wirkstoff über diese NH- oder OH-Gruppe mit der Verbindung der Formel 1 verestert bzw. amidiert ist. Die Bindung zwischen dem Benzoxazindionderivat und dem Antibiotikum kann sowohl direkt als auch über gebräuchliche Linkergruppen, z. B. Aminocarbonsäuren, Hydroxycarbonsäuren, Diamine oder Diole erfolgen. Unter einem Antibiotikum ist ein entsprechendes eine NH-oder OH-Gruppe enthaltenes β-Lactam, wie z. B. ein Cephalosporin, z. B. Cephalexin, Cephadroxil oder Claforan, oder ein Penicillin, z. B. Ampicillin, Amoxicillin oder ein O-Acyl-amoxicillinderivat, oder ein Tetracyclinderivat, z. B. ein Aminodoxycyclin, oder ein Antibiotikum vom Typ der Aminoglykoside, Makrolide, Chinolone oder Carbapeneme zu verstehen.

Im Falle des Vorliegens asymmetrischer C-Atome sind die entsprechenden D- und L-Formen, Enantiomere und Diastereomere sowie die Racemate bzw. Enantiomeren- und Diastereomerengemische ebenfalls Gegenstand der Erfindung.

Die genannten Verbindungen können als freie Säuren, in Form ihrer Salze oder als leicht spaltbare, wie unter physiologischen Bedingungen spaltbare Ester vorliegen.

Zur Herstellung der erfindungsgemäßen Verbindungen der Formel l werden zunächst Verbindungen der Formel **l** mit R⁵ oder R⁹ = OH hergestellt durch Kondensation von 2,3-Diacyloxybenzoylchlorid mit entsprechenden Aminokomponenten, z. B. Aminosäuren, Dipeptiden oder Aminobenzoesäuren in Natriumbicarbonatlösung entsprechend dem angegebenen Formelschema 1: 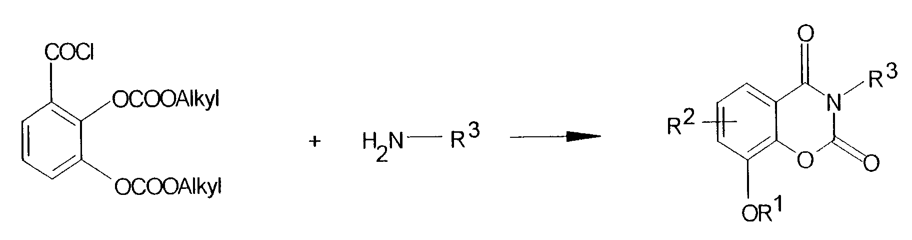 mit R¹ = COOAlkyl oder H

### Formelschema 1

Im Verlauf der Reaktion kann die COOAlkyl-Gruppe an R¹ hydrolytisch gespalten werden, so daß Verbindungen mit R¹ = H entstehen können. Diese können erneut acyliert werden, z. B. zu Verbindungen mit R¹ = COAlkyl.

Verbindungen der Formel **l** mit R⁵ oder R⁹ = OH können nach demselben Prinzip auch nach dem Reaktionsschema **2** hergestellt werden, indem entsprechende Amidderivate, z. B. 2,3-Dihydroxybenzhydrazone, mit Chlorameisensäurealkylestern in alkalischem Milieu umgesetzt werden.

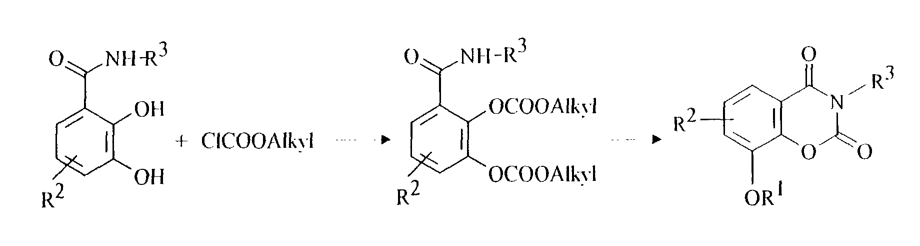

### Formelschema 2

Die erfindungsgemäßen Verbindungen der Formel **l** mit R³, R⁵, R⁹ bzw. X = ein Wirkstoffrest wie oben definiert, werden z. B. hergestellt, indem
a. zunächst aus einer Verbindung der Formel **l** mit A = H nach üblichen Verfahren, z. B. mittels Phosphorpentachlorid in Tetrachlorkohlenstoff, das entsprechende Chlorid hergestellt wird und dieses dann mit einem Wirkstoff wie oben definiert, welcher eine freie OH- oder NH-Gruppe sowie gegebenenfalls eine gebräuchliche Linkergruppe, wie z. B. Reste einer Diaminocarbonsäure, einer Hydroxycarbonsäure bzw. eines Diamins oder Diols enthält, in einem geeigneten Lösungsmittel, z. B. Tetrahydrofuran, umgesetzt wird, oder indem
b. eine Verbindung der Formel **l** mit A = H z. B. nach der Gemischtanhydridmethode zunächst mit Chlorameisensäureester und einem tertiären Amin, z. B. Triethylamin, und dann mit dem entsprechenden oben definierten Wirkstoff, der eine freie NH- oder OH-Gruppe sowie gegebenenfalls eine gebräuchliche Linkergruppe, wie z. B. Reste einer Diaminocarbonsäure, einer Hydroxycarbonsäure bzw. eines Diamins oder Diols, enthält, zusammen mit einem geeigneten tertiären Amin, z. B. Triethylamin in einem geeigneten Lösungsmittel, z. B. Tetrahydrofuran, umgesetzt wird.

Die Verbindungen der Formel **l** mit einer Carboxylgruppe können als freie Säuren, in Form ihrer Salze oder als leicht spaltbare, insbesondere unter physiologischen Bedingungen spaltbare, Ester vorliegen. Die Reinigung der Verbindungen erfolgt nach üblichen, aus dem Stand der Technik bekannten Verfahren, beispielsweise durch Umkristallisation oder mittels chromatographischer Methoden.

Die erfindungsgemäßen Verbindungen der Formel **l** zeigen bei verschiedenen gramnegativen Bakterienstämmen Siderophorwirksamkeit. Infolgedessen können diese Verbindungen als Wachstumsfaktoren für bestimmte Bakterienkulturen angewandt werden.

Die Prüfung auf Siderophorwirksamkeit gemäß DIN 58 940 erfolgte mit verschiedenen bakteriellen Indikatormutanten, die aufgrund fehlender eigener Fisentransportsysteme unter den Testbedingungen kein Wachstum zeigen. Nach Zugabe der Testsubstanzen als Fremdsiderophore kann eine Wachstumsförderung festgestellt werden. Bei den Indikatormutanten ist die Biosynthese der jeweiligen Siderophore, z. B. Pyoverdin, Pyochelin, Enterobactin, Aerobactin, Yersiniabactin oder z. B. die Aromatenbiosynthese geblockt oder es fehlen Rezeptoren für Enterobactin, Pyochelin bzw. Pyoverdin sowie andere wichtige Komponenten für den bakteriellen Eisentransport (z. B. Membranproteine Cir, Fiu, FepA, auch das TonB-Protein). Diese Mutanten können deshalb unter Eisenmangelbedingungen nicht oder nur sehr verzögert wachsen. Im einzelnen wurden folgende Indikatormutanten verwendet: *Pseudomonas aeruginosa:* PAO 6609, K 407, *E.coli:* AB 2847, *Salmonella typhimurium:* enb-7, TA 2700, *Yersinia enterocolitica* WAH 5030. Als Kontrollen wurden bei den Pseudomonas-Stämmen und Y. *enterocolitica* Ferrioxamin E, bei den *Salmonella-Stämmen* Ferrioxamin G und Enterobactin, bei den E.coli-Stämmen Ferrichrom eingesetzt. Die Testsubstanzen wurden in einer Menge von jeweils 5 µg/Testblättchen appliziert.

Die Wachstumszonen der Indikatormutanten (Durchmesser in mm) unter dem Einfluß der Testsubstanzen sind in der Tabelle 1 angegeben.

Aufgrund ihrer Eigenschaften als bakterielle Siderophore können die Verbindungen der allgemeinen Formel **l** als Transportvehikel bzw. Penetrationsbeschleuniger für antimikrobielle Antibiotika und andere Wirkstoffe dienen, d.h. sie können als Konjugate mit Antibiotika bzw. anderen Wirkstoffen diese über Eisentransportwege in die Mikrobenzelle transportieren und somit deren Wirksamkeit erhöhen. Infolgedessen besitzen Verbindungen der allgemeinen Formel **l** mit R³, R⁵, R⁹ bzw. X = ein Wirkstoffrest, speziell ein β-Lactam, antibakterielle Wirksamkeit, z.T. auch bei gegenüber anderen ß-Lactamen resistenten Bakterien, wobei die vorhandene Siderophorwirkung des Benzoxazindionrestes durch die antibakterielle Wirkung des Gesamtmoleküls überdeckt wird. Für die Bestimmung der antibakteriellen Wirksamkeit wurden in einem Mikrodilutionstest gemäß DIN 58 940 die minimalen Hemmkonzentrationen (MHK) bei folgenden Bakterienstämmen ermittelt: *Pseudomonas* aeruginosa SG 137, NCTC 10662, ATCC 27853, *E.coli* ATCC 25922, *Klebsiella pneumoniae* ATCC 10031, *Stenotrophomonas maltophilia GN* 12873 und *Staphylococcus aureus* SG 511. Die Ergebnisse des Tests sind in Tabelle 2 angegeben. Danach zeigen die getesteten Verbindungen hohe antibakterielle Wirksamkeit, die z. T. die der Vergleichssubstanzen Azlocillin und Ampicillin übertrifft. Durch Variation des Eisengehaltes der Testmedien und durch Einsatz von Eisentransportmutanten wurde die Abhängigkeit der antibakteriellen Wirkung vom bakteriellen Eisentransport nachgewiesen.

Verbindungen der allgemeinen Formel **l** mit R³, R⁵, R⁹ bzw. X = ein Wirkstoffrest wie oben definiert sowie beim Vorliegen saurer Gruppen auch deren Salze und unter physiologischen Bedingungen spaltbare Ester, eignen sich aufgrund ihrer antibakteriellen Wirksamkeit zur Anwendung als Arzneimittel gegen bakterielle Infektionen bei Menschen und Nutztieren.

Bei den genannten Erkrankungen können die Verbindungen der Formel l entweder allein oder in Form von pharmazeutischen Präparaten mit physiologisch verträglichen, aus dem Stand der Technik bekannten Hilfs- oder Trägerstoffen angewandt werden, wobei prinzipiell alle üblichen pharmakologischen Anwendungsformen möglich sind.

### Beispiele

### Beispiel 1

Herstellung von (8-Methoxycarbonyloxy-2,4-dioxo-benzoxazin-3-yl)-essigsäure (Formel I mit R¹ = COOCH₃, R² = H, R³ = CH₂COOH)

Eine Lösung von 2,75 g Glycin in 175 ml 0,5M Natriumhydrogencarbonatlösung wurde im Ultraschallbad auf 0-5° C gekühlt. Unter Rühren wurden bei 0 - 5°C 10,5 g 2,3-Di-(methoxycarbonyloxy)-benzoylchlorid, in 20 ml absolutem Tetrahydrofuran gelöst, zugetropft. Die nach 1 Stunde entstandene trübe Lösung wurde filtriert und das Tetrahydrofuran abdestilliert. Die erhaltene Substanz wurde abgesaugt und mit wenig kaltem Wasser gewaschen. Zur Reinigung wurde die Substanz erneut in 0,5 M Natriumhydrogencarbonatlösung gelöst, filtriert und mit konzentrierter Salzsäure ausgefällt. Es wurden farblose Kristalle mit einem Schmelzpunkt von 205-208°C in einer Ausbeute von 70% der Theorie erhalten.

### Beispiel 2

Herstellung von (8-Methoxycarbonyloxy-2,4-dioxo-benzoxazin-3-yl)-acetylglycin (Formel I mit R¹ =COOCH₃, R² = H, R³ = CH₂CONHCH₂COOH).

Die Verbindung wurde analog zu Beispiel 1 aus Glycylglycin und 2,3-Di-(methoxycarbonyloxy)-benzoylchlorid in wäßriger Natriumbicarbonatlösung hergestellt. Es wurden farblose Kristalle mit einem Schmelzpunkt von 195-198°C nach Rekristallisation aus Wasser in einer Ausbeute von 70% der Theorie erhalten.

### Beispiel 3

Herstellung von (8-Methoxycarbonyloxy-2,4-dioxo-benzoxazin-3-yl)-acetyl-L-alanin (Formel I mit R¹ = COOCH₃, R² = H, R³=CH₂CONH-CH(CH₃)-COOH).

Die Verbindung wurde analog zu Beispiel 1 aus Glycyl-L-alanin und 2,3-Di-(methoxycarbonyloxy)-benzoylchlorid in wäßriger Natriumbicarbonatlösung hergestellt. Es wurden farblose Kristalle mit einem Schmelzpunkt von 180-185°C nach Rekristallisation aus Essigsäureethylester in einer Ausbeute von 70% der Theorie erhalten.

### Beispiel 4

Herstellung von (8-Hydroxy-2,4-dioxo-benzoxazin-3-yl)-acetyl-L-alanin (Formel I mit R¹ = H, R² = H, R³=CH₂CONH-CH(CH₃)-COOH).

Die Verbindung ließ sich aus der bei der Isolierung von (8-Methoxycarbonyloxy-2,4-dioxo-benzoxazin-3-yl)-acetyl-L-alanin (Produkt aus Beispiel 3) anfallenden Mutterlauge durch präparative HPLC (Elutionsmittel: Acetonitril/Wasser = 1/1 mit 0,05 % Trifluoressigsäure) gewinnen. Es wurden farblose Kristalle mit einem Schmelzpunkt von 203-204 °C nach Rekristallisation aus Essigsäureethylester in einer Ausbeute von 20% der Theorie erhalten.

### Beispiel 5

Herstellung von (8-Methoxycarbonyloxy-2,4-dioxo-benzoxazin-3-yl)-acetyl-L-leucin (Formel I mit R¹ = COOCH₃, R² = H, R³=CH₂CONH-CH(COOH)-CH₂CH(CH₃)₂) Die Verbindung wurde analog zu Beispiel 1 aus Glycylleucin und 2,3-Di-(methoxycarbonyloxy)-benzoylchlorid in wäßriger Natriumbicarbonatlösung hergestellt. Die Isolierung erfolgte durch sofortige Extraktion des nach Ansäuern mit Salzsäure erhaltenen Gemisches mit Essigsäureethylester, mehrfachem Waschen der so erhaltenen Lösung mit Wasser, Trocknen mit Natriumsulfat und Entfernen des Lösungsmittels im Vakuum. Nach präparativer HPLC (Elutionsmittel: Acetonitril/Wasser = 1/1 mit 0,05 % Trifluoressigsäure) und Rekristallisaton aus Wasser wurden farblose Kristalle mit einem Schmelzpunkt von 179-181° C in einer Ausbeute von 60% der Theorie erhalten.

### Beispiel 6

Herstellung von (8-Hydroxy-2,4-dioxo-benzoxazin-3-yl)-acetyl-L-leucin (Formel I mit R¹ = H, R² = H, R³=CH₂CONH-CH(COOH)-CH₂CH(CH₃)₂)

Die Verbindung wurde mittels präparativer HPLC (Elutionsmittel: Acetonitril/Wasser = 1/1 mit 0,05 % Trifluoressigsäure) als zweites Produkt aus dem bei der Herstellung von (8-Methoxycarbonyloxy-2,4-dioxo-benzoxazin-3-yl)-acetyl-L-leucin (Produkt aus Beispiel 5) erhaltenen Reaktionsgemisch isoliert. Nach Rekristallisation aus Essigsäureethylester wurden farblose Kristalle mit einem Schmelzpunkt von 204-207°C in einer Ausbeute von 25% der Theorie erhalten.

### Beispiel 7

Herstellung von 2-L-(8-Methoxycarbonyloxy-2,4-dioxo-benzoxazin-3-yl)-propionsäure (Formel I mit R¹ =COOCH₃, R² = H, R³ = CH(CH₃)-COOH)

Die Verbindung wurde analog zu Beispiel 4 aus L-Alanin und 2,3-Di-(methoxycarbonyloxy)-benzoylchlorid in wäßriger Natriumbicarbonatlösung hergestellt. Nach präparativer HPLC (Elutionsmittel: Acetonitril/Wasser = 2/3 mit 0,05 % Trifluoressigsäure) wurde ein farbloser Schaum in einer Ausbeute von 50% der Theorie erhalten.

### Beispiel 8

Herstellung von L-(8-Methoxycarbonyloxy-2,4-dioxo-benzoxazin-3-yl)-phenylessigsäure (Formel I mit R¹ =COOCH₃, R² = H, R³ =C₆H₅-CH-COOH) Die Verbindung wurde analog zu Beispiel 4 aus L-Phenylalanin und 2,3-Di-(methoxycarbonyloxy)-benzoylchlorid in wäßriger Natriumbicarbonatlösung hergestellt. Nach präparativer HPLC (Elutionsmittel: Acetonitril/Wasser = 1/1 mit 0,05 % Trifluoressigsäure) und Rekristallisation aus Wasser wurden farblose Kristalle mit einem Schmelzpunkt von 182 - 184°C in einer Ausbeute von 50% der Theorie erhalten.

### Beispiel 9

Herstellung von 4-[(8-Methoxycarbonyloxy-2,4-dioxo-benzoxazin-3-ylimino)-methyl]-benzoesäure (Formel l mit R¹ = COOCH₃, R² = H, R³ = -N=CH-C₆H₄-COOH(p)

Zu einer Lösung von 0,3 g 4-[(2,3-Dihydroxybenzoyl)-hydrazonomethyl)]-benzoesäure in 2ml 2N Natronlauge und 3 ml Wasser wurden unter Rühren bei 0°C 2 ml Chlorameisensäuremethylester zugegeben. Die Mischung wurde 30 Minuten gerührt und dann mit Salzsäure angesäuert. Das Rohprodukt wurde in heißem Dimethylformamid gelöst, die Lösung filtriert und das Produkt mit Wasser wieder ausgefällt. Die weitere Reinigung erfolgte mittels präparativer HPLC (Elutionsmittel: Acetonitril/Wasser = 1/1 mit 0,05 % Trifluoressigsäure). Es wurden farblose Kristalle mit einem Schmelzpunkt von 232-234 °C erhalten.

### Beispiel 10

Herstellung von N-[(8-Methoxycarbonyloxy-2,4-dioxo-benzoxazin-3-yl)-acetyl]-ampicillin (Formel I mit R¹ =COOCH₃, R² = H, R³ = CH₂-CO-R⁵, R⁵ = N-Ampicillino-)
(a) Herstellung von (8-Methoxycarbonyloxy-2,4-dioxo-benzoxazin-3-yl)-acetylchlorid.
   Eine Mischung von 1,07 g (3mmol) 8-Methoxycarbonyloxy-2,4-dioxo-benzoxazin-3-yl-essigsäure (Substanz 1) und 1 g Phosphorpentachlorid in 5 ml absolutem Tetrachlorkohlenstoff wurde vorsichtig bis zur Beendigung der HCI-Entwicklung (30 Minuten) erwärmt. Die entstandene Lösung wurde filtriert und das Filtrat eingeengt. Der Rückstand wurde erneut in heißem Tetrachlorkohlenstoff gelöst, das Säurechlorid mit wasserfreiem Petrolether ausgefällt und im Hochvakuum getrocknet. Es wurden so 0,81 g des Produkts (86% der Theorie) mit einem Schmelzpunkt von 80 - 82°C erhalten.
(b) Eine Lösung von 0,78 g Ampicillin-Natriumsalz in 12 ml 80%igem Tetrahydrofuran wurde auf -5°C gekühlt. Dazu wurden unter Rühren portionsweise 0,63 g 8-Methoxycarbonyloxy-2,4-dioxo-benzoxazin-3-yl-acetylchlorid zugegeben. Die Mischung wurde 1 Stunde bei 0°C und 1 Stunde bei 20° C gerührt, dann im Vakuum eingedampft. Der Rückstand wurde mit 50 ml Wasser und 50 ml Essigsäureethylester versetzt. Dann wurde mit 1M Salzsäure schwach angesäuert (pH 3) und dreimal mit wäßriger Natriumchloridlösung gewaschen. Die organische Phase wurde abgetrennt, über Natriumsulfat getrocknet und auf ein Volumen von 20 ml eingeengt. Der Rückstand wurde mit Petrolether versetzt, wobei 0,92 g (74 % der Theorie) des Ampicillinderivats als weißes Pulver anfielen.

### Beispiel 11

Herstellung von N-[(8-Methoxycarbonyloxy-2,4-dioxo-benzoxazin-3-yl)-acetyl]-amoxicillin (Formel I mit R¹ = COOCH₃, R² = H, R³=CH₂COR⁵, R⁵ = N-Amoxicillino-)

Eine Lösung von 0,55 g Amoxicillin in 8 ml 80%igem Tetrahydrofuran wurde mit 0,22 ml Triethylamin versetzt und auf -5°C gekühlt. Dazu wurden unter Rühren portionsweise 0,45 g (8-Methoxycarbonyloxy-2,4-dioxo-benzoxazin-3-yl)-acetylchlorid (Herstellung gemäß Beispiel 8) zugegeben. Die Mischung wurde je 1 Stunde bei 0°C und bei 20° C gerührt und dann im Vakuum eingedampft. Der Rückstand wurde mit 40 ml Wasser und 40 ml Essigsäureethylester versetzt. Es wurde mit 1 M Salzsäure schwach angesäuert (pH 3), geschüttelt und mit wäßriger Natriumchloridlösung säurefrei gewaschen. Die abgetrennte organische Phase wurde über Natriumsulfat getrocknet, weitgehend eingedampft und mit Petrolether versetzt. Dabei fiel das Amoxicillinderivat als weißes Pulver mit einer Ausbeute von 80 % der Theorie an.

### Beispiel 12

Herstellung von N-[(8-Methoxycarbonyloxy-2,4-dioxo-benzoxazin-3-yl)-acetyl-glycyl]-ampicillin (Formel I mit R¹ =COOCH₃, R² = H, R³ = CH₂-CO-NH-CH₂-COR⁵ R⁵ = N-Ampicillino-)

0.352 g (8-Methoxycarbonyloxy-2,4-dioxo-benzoxazin-3-yl)-acetyl-glycin (Herstellung siehe Beispiel 2), 140 µl Triethylamin und eine katalytische Menge Dimethylaminopyridin wurden in 5 ml absolutem Tetrahydrofuran gelöst und die Lösung bei -20°C mit 126 µl Chlorameisensäureisobutylester unter Rühren versetzt. Die Mischung wurde 30 Minuten bei -20° C gerührt. Anschließend wurden 0,357 g Ampicillin-Natriumsalz in 5ml 80%igem Tetrahydrofuran zugegeben. Es wurde je 1 Stunde bei -20°C und bei +20° C gerührt, dann im Vakuum eingedampft. Der Rückstand wurde mit Essigsäureethylester und Wasser versetzt und vorsichtig mit 1M Salzsäure angesäuert. Die organische Phase wurde abgetrennt, dreimal mit Natriumchloridlösung gewaschen, über Natriumsulfat getrocknet und teilweise eingedampft. Durch Zusatz von Petrolether wurde das Ampicillinderivat ausgefällt, das mittels präparativer HPLC (Elutionsmittel: Acetonitril/ Wasser = 1/1 mit 0,05 % Trifluoressigsäure) gereinigt wurde.

### Beispiel 13

Herstellung von N-[(8-Methoxycarbonyloxy-2,4-dioxo-benzoxazin-3-yl)-acetyl-L-alanyl]-ampicillin (Formel I mit R¹ = COOCH₃, R² = H, R³ =CH₂-CONH-CH(CH₃)-COR⁵, R⁵ = N-ampicillino-)

Die Herstellung der Verbindung erfolgte analog zu Beispiel 12 aus (8-Methoxycarbonyloxy-2,4-dioxo-benzoxazin-3-yl)-acetyl-L-alanin (Herstellung siehe Beispiel 3) und Ampicillin-Natriumsalz. Die Reinigung erfolgte mittels präparativer HPLC (Elutionsmittel: Acetonitril/Wasser = 1/1 mit 0,05 % Trifluoressigsäure), wobei ein farbloser Feststoff mit einer Ausbeute von 65% der Theorie erhalten wurde.

### Beispiel 14

Herstellung von N-[(8-Methoxycarbonyloxy-2,4-dioxo-benzoxazin-3-yl)-acetyl-L-leucyl]-ampicillin (Formel I mit R¹ =COOCH₃, R² = H, R³ = CH₂-CONH-CH(COR⁵)-CH₂CH(CH₃)₂, R⁵ = N-ampicillino-)

Die Verbindung wurde analog zu Beispiel 12 aus (8-Methoxycarbonyloxy-2,4-dioxo-benzoxazin-3-yl)-acetyl-L-leucin (Herstellung siehe Beispiel 5) und Ampicillin-Natriumsalz hergestellt. Die Reinigung erfolgte mittels präparativer HPLC (Elutionsmittel: Acetonitril/Wasser = 2/3 mit 0,05 % Trifluoressigsäure), wobei ein farbloser Feststoff in einer Ausbeute von 60% der Theorie anfiel.

Beispiel 15

### Herstellung von N-[(8-Methoxycarbonyloxy-2,4-dioxo-benzoxazin-3-yl)-ace-tyl]-O-n-propionyl-amoxicillin (Formel I mit R¹ = COOCH₃, R² = H, R³ = CH₂COR⁵, R⁵ = N-(O-n-Propionyl)-amoxicillino-)

0,385 g N-[(8-Methoxycarbonyloxy-2,4-dioxo-benzoxazin-3-yl)-acetyl]-amoxicillin (Herstellung siehe Beispiel 11) wurden in 25 ml Tetrahydrofuran gelöst und die Lösung auf -78°C gekühlt. Unter Rühren wurden zunächst 0,34 ml Triethylamin, dann 0,16 ml Propionylchlorid zugefügt. Die Reaktionsmischung wurde 30 Minuten bei -60°C und 1 Stunde bei 20° C gerührt. Danach wurde im Vakuum eingedampft und der Rückstand mit Wasser und Essigsäureethylester versetzt. Nach dem Ansäuern mit 1M Salzsäure (pH 3) wurde mit wäßriger Natriumchloridlösung säurefrei gewaschen. Die abgetrennte organische Phase wurde über Natriumsulfat getrocknet, weitgehend eingedampft und mit Petrolether versetzt. Dabei fiel das Amoxillinderivat als weißes Pulver in einer Ausbeute von 40% der Theorie an.

### Beispiel 16

Herstellung von 4-(8-Methoxycarbonyioxy-2,4-dioxo-benzoxazin-3-yl)-benzoesäure (Formel I mit R¹ = COOCH₃, R² = H, R³ = C₆H₄-COOH (p))

Die Verbindung wurde analog zu Beispiel 1 aus 4-Aminobenzoesäure und 2,3-Di-(methoxycarbonyloxy)-benzoylchlorid in wäßriger Natriumhydrogencarbonatlösung hergestellt. Es wurden nach Rekristallisation aus Essigsäureethylester farblose Kristalle mit einem Schmelzpunkt von 236 - 240°C in einer Ausbeute von 80% der Theorie erhalten.

### Beispiel 17

Herstellung von 2L,6-Bis(8-methoxycarbonyloxy-2,4-dioxo-1,3-benzoxazin-3-yl)-hexansäure (Formel I mit R¹ = COOCH₃, R² = H, R³ = CHR⁴-COOH, R⁴ = (CH₂)₄Y)

Die Verbindung wurde analog zu Beispiel 5 aus L-Lysin und 2 Moläquivalenten 2,3-Di-(methoxycarbonyloxy)-benzoylchlorid in wäßriger Natriumhydrogencarbonatlösung als farbloser Schaum erhalten.

### Beispiel 18

Herstellung von N-[4-(8-Methoxycarbonyloxy-2,4-dioxo-1,3-benzoxazin-3-yl)- benzoyl]-ampicillin Natriumsalz (Formel I mit R¹ = COOCH₃, R² = H, R³ = R²⁰ mit R¹³ = H, COR⁹ in 4-Position, R⁹ = N-Ampicillino (Na-Salz), p = 0)
(a) Herstellung von 4-(8-Methoxycarbonyloxy-2,4-dioxo-1,3-benzoxazin-3-yl)-benzoylchlorid
   Die Verbindung wurde analog zu Substanz 10a aus Substanz 16 und Phosphorpentachlorid in Tetrachlorkohlenstoff hergestellt. Erhalten wurde ein farbloses Öl in einer Ausbeute von 65% der Theorie.
(b) Herstellung von N-[4-(8-Methoxycarbonyloxy-2,4-dioxo-1,3-benzoxazin-3-yl)-benzoyl]-ampicillin
   Die Verbindung wurde analog zu Substanz 10b aus 4-(8-Methoxycarbonyloxy-2,4-dioxo-1,3-benzoxazin-3-yl)-benzoylchlorid und Ampicillin-Natriumsalz hergestellt. Es wurde ein weißes Pulver in einer Ausbeute von 85% der Theorie erhalten.
(c) Natriumsalz: Eine Lösung von 0,25 g N-[4-(8-Methoxycarbonyloxy-2,4-dioxo-1,3-benzoxazin-3-yl)-benzoyl]-ampicillin in Essigsäureethylester wurde unter Eiskühlung und Rühren mit einer Lösung von 0,083 g Natrium-2-ethylhexanoat in Essigsäureethylester versetzt. Nach Ausfällung des Produktes mit Petrolether wurde abfiltriert. Die Reinigung erfolgte mittels präparativer HPLC (Elutionsmittel: Acetonitril/Wasser). Hierbei wurde ein weißes Pulver in einer Ausbeute von 0,202 g (79% der Theorie) erhalten.

### Beispiel 19

Herstellung von 4-(8-Methoxycarbonyloxy-2,4-dioxo-1,3-benzoxazin-3-yl-methyl)-benzoesäure (Formel I mit R¹ = COOCH₃, R² = H, R³ = R²⁰ mit R¹³ = H, COR⁹ in 4-Position, R⁹ = OH, p = 1)

Die Verbindung wurde analog zu Substanz 5 aus 4-(Aminomethyl)-benzoesäure und 2,3-Di-(methoxycarbonyloxy)-benzoylchlorid in wäßriger Natriumhydrogencarbonatlösung hergestellt. Nach Umkristallisation aus Essigsäureethylester wurden farblose Kristalle mit einem Schmelzpunkt von 220-222°C in einer Ausbeute von 65% der Theorie erhalten.

### Beispiel 20

### Herstellung von N-[4-(8-Methoxycarbonyloxy-2,4-dioxo-1,3-benzoxazin-3-yl-methyl)-benzoyl]-ampicillin Natriumsalz (Formel I mit R¹ COOCH₃, R² = H, R³ = R²⁰ mit R¹³ = H, COR⁹ in 4-Position, R⁹ = N-Ampicillino (Na-Salz), p = 1)

(a) Herstellung von 4-(8-Methoxycarbonyloxy-2,4-dioxo-1,3-benzoxazin-3-yl-methyl)-benzoylchlorid
   Die Verbindung wurde analog zu Substanz 10a aus Substanz 19 und Phosphorpentachlorid in Tetrachlorkohlenstoff hergestellt. Erhalten wurde ein farbloses Öl in einer Ausbeute von 95% der Theorie.
(b) Herstellung von N-[4-(8-Methoxycarbonyloxy-2,4-dioxo-1,3-benzoxazin-3-yl-methyl)-benzoyl]-ampicillin
   Die Verbindung wurde analog zu Substanz 10b aus 4-(8-Methoxycarbonyl-oxy-2,4-dioxo-1,3-benzoxazin-3-yl-methyl)-benzoylchlorid und Ampicillin-Natriumsalz hergestellt. Erhalten wurde ein weißes Pulver in einer Ausbeute von 80% der Theorie.
(c) Natriumsalz: Die Herstellung erfolgte analog zu Substanz 18 aus N-[4-(8-Methoxycarbonyloxy-2,4-dioxo-1,3-benzoxazin-3-yl-methyl)-benzoyl]-ampicillin und Natrium-2-ethylhexanoat in Essigsäureethylester. Erhalten wurde ein weißes Pulver in einer Ausbeute von 60% der Theorie.

### Beispiel 21

Herstellung von 3,5-Bis-(8-methoxycarbonyloxy-2,4-dioxo-1,3-benzoxazin-3-yl)-benzoesäure (Formel I mit R¹ = COOCH₃, R² = H, R³ = R²⁰ mit R¹³ = Y in 3-Position, COR⁹ in 5-Position, R⁹ = OH, p = 0, R¹, R² wie oben)

Die Verbindung wurde analog zu Substanz 5 aus 3,5-Diaminobenzoesäure und 2 Moläquivalenten 2,3-Di-(methoxycarbonyloxy)-benzoylchlorid in wäßriger Natriumhydrogencarbonatlösung hergestellt. Nach Umkristallisation aus Essigsäureethylester wurden farblose Kristalle mit einem Schmelzpunkt von 164-166°C in einer Ausbeute von 65% der Theorie erhalten.

### Beispiel 22

Herstellung von N-[3,5-Bis-(8-methoxycarbonyloxy-2,4-dioxo-1,3-benzoxazin-3-yl)-benzoyl]-ampicillin Natriumsalz (Formel I mit R¹ = COOCH₃, R² = H, R³ = R²⁰ mit R¹³ = Y in 3-Position, COR⁹ in 5-Position, R⁹ = -N-Ampicillino (Na-Salz), p = 0, R¹, R² wie oben)
(a) Herstellung von 3,5-Bis-(8-methoxycarbonyloxy-2,4-dioxo-1,3-benzoxazin-3-yl)-benzoylchlorid
   Die Verbindung wurde analog zu Substanz 10a aus Substanz 21 und Phosphorpentachlorid in Tetrachlorkohlenstoff hergestellt. Sie wurde als farbloser Schaum in einer Ausbeute von 90% der Theorie erhalten.
(b) Herstellung von N-[3, 5-Bis-(8-methoxycarbonyloxy-2,4-dioxo-1,3-benzoxazin-3-yl)-benzoyl]-ampicillin
   Die Verbindung wurde analog zu Substanz 10b aus 3,5-Bis-[(8-methoxycarbonyloxy-2,4-dioxo-1,3-benzoxazin-3-yl)-benzoylchlorid und Ampicillin-Natriumsalz hergestellt. Sie wurde als weißes Pulver in einer Ausbeute von 80% der Theorie erhalten.
(c) Natriumsalz: Die Verbindung wurde analog zu Substanz 18 aus N-[3,5-Bis-(8-methoxycarbonyloxy-2,4-dioxo-1,3-benzoxazin-3-yl)-benzoyl]-ampicillin und Natrium-2-ethylhexanoat in Essigsäureethylester hergestellt. Erhalten wurde ein weißes Pulver in einer Ausbeute von 40% der Theorie.

### Beispiel 23

Herstellung von 3-(8-Methoxycarbonyloxy-2,4-dioxo-1,3-benzoxazin-3-yl)-propionsäure (Formel I mit R¹ = COOCH₃, R² = H, R³ = (CH₂)₂COOH)

Die Verbindung wurde analog zu Substanz 5 aus β-Alanin und 2,3-Di-(methoxycarbonyloxy)-benzoylchlorid in wäßriger Natriumhydrogencarbonatlösung hergestellt. Nach Umkristallisation aus Essigsäureethylester wurden farblose Kristalle mit einem Schmelzpunkt von 140-144°C in einer Ausbeute von 55% der Theorie erhalten.

### Beispiel 24

Herstellung von N-[3-(8-Methoxycarbonyloxy-2,4-dioxo-1,3-benzoxazin-3-yl)-propionyl]-ampicillin Natriumsalz (Formel I mit R¹ = COOCH₃, R² = H, R³ = (CH₂)₂CO-N-Ampicillino (Na-Salz))
(a) Herstellung von 3-(8-Methoxycarbonyloxy-2,4-dioxo-1,3-benzoxazin-3-yl)-propionylchlorid
   Die Verbindung wurde analog zu Substanz 10a aus Substanz 23 und Phosphorpentachlorid in Tetrachlorkohlenstoff hergestellt. Erhalten wurde ein gelbliches Öl in einer Ausbeute von 100% der Theorie.
(b) Herstellung von N-[3-(8-Methoxycarbonyloxy-2,4-dioxo-1,3-benzoxazin-3-yl)-propionyl]-ampicillin
   Die Verbindung wurde analog zu Substanz 10b aus 2-(8-Methoxycarbonyloxy-2,4-dioxo-1,3-benzoxazin-3-yl)-propionylchlorid und Ampicillin-Natriumsalz hergestellt. Erhalten wurde ein weißes Pulver in einer Ausbeute von 88% der Theorie.
(c) Natriumsalz: Das Salz wurde analog zu Substanz 18 aus N-[3-(8-Methoxycarbonyloxy-2,4-dioxo-1,3-benzoxazin-3-yl)-propionyl]-ampicillin und Natrium-2-ethylhexanoat in Essigsäureethylester hergestellt. Erhalten wurde ein weißes Pulver in einer Ausbeute von 41% der Theorie.

### Beispiel 25

Herstellung von 3,5-Bis-[3-(8-methoxycarbonyloxy-2,4-dioxo-1,3-benzoxazin-3-yl)-propionylamino]-benzoesäure (Formel I mit R¹ = COOCH₃, R² = H, R³ = R¹⁸ mit R¹⁰, R¹¹ = H, R¹² = R¹⁹ in 3-Position, COR⁹ in 5-Position, R⁹ = OH, R¹⁵, R¹⁴ = H, n = 2, R¹, R² wie oben)

Die Verbindung wurde analog zu Substanz 5 aus 3,5-Diaminobenzoesäure und 2 Moläquivalenten 3-(8-Methoxycarbonyloxy-2,4-dioxo-1,3-benzoxazin-3-yl)-propionylchlorid (Substanz 24a) in wäßriger Natriumhydrogencarbonatlösung hergestellt. Erhalten wurden farblose Kristalle mit einem Schmelzpunkt von 160-165°C in einer Ausbeute von 50% der Theorie.

### Beispiel 26

Herstellung von N-{3,5-Bis-[3-(8-methoxycarbonyloxy-2,4-dioxo-1,3-benzoxazin-3-yl)-propionylamino]-benzoyl}-ampicillin Natriumsalz (Formel I mit R¹ = COOCH₃, R² = H, R³ = R¹⁸ mit R¹⁰, R¹¹ = H, R¹² = R¹⁹ in 3-Position, COR⁹ in 5-Position, R⁹ = N-Ampicillino (Na-Salz), R¹⁵, R¹⁴ = H, n = 2, R¹, R² wie oben)
(a) Herstellung von N-{3,5-Bis-[3-(8-methoxycarbonyloxy-2,4-dioxo-1,3-benzoxazin-3-yl)-propionylamino]-benzoyl}-ampicillin
   Die Verbindung wurde analog zu Substanz 12 aus Substanz 23 und Ampicillin-Natriumsalz hergestellt. Erhalten wurde ein weißes Pulver in einer Ausbeute von 80% der Theorie.
(b) Natriumsalz: Die Verbindung wurde analog zu Substanz 18 aus N-{3,5-Bis-[3-(8-methoxycarbonyloxy-2,4-dioxo-1,3-benzoxazin-3-yl)-propionylamino]-benzoyl}-ampicillin und Natrium-2-ethylhexanoat in Essigsäureethylester hergestellt. Erhalten wurde ein weißes Pulver in einer Ausbeute von 18% der Theorie.

### Beispiel 27

Herstellung von 3,5-Bis-[(8-methoxycarbonyloxy-2,4-dioxo-1,3-benzoxazin- n-3-yl)-acetylamino]-benzoesäure (Formel I mit R¹ = COOCH₃, R² = H, R³ = R¹⁸ mit R¹⁰, R¹¹ = H, R¹² = R¹⁹ in 3-Position, COR⁹ in 5-Position, R⁹ = OH, R¹⁵, R¹⁴ = H, n = 1, R¹, R² wie oben)

Die Verbindung wurde analog zu Substanz 5 aus 3,5-Diaminobenzoesäure und 2 Moläquivalenten (8-Methoxycarbonyloxy-2,4-dioxo-1,3-benzoxazin-3-yl)-acetylchlorid (Substanz 10a) in wäßriger Natriumhydrogencarbonatlösung hergestellt. Nach Umkristallisation aus Essigsäureethylester wurden farblose Kristalle mit einem Schmelzpunkt von 190-195°C in einer Ausbeute von 53% der Theorie erhalten.

### Beispiel 28

Herstellung von N-{3, 5-Bis-[(8-methoxycarbonyloxy-2,4-dioxo-1,3-benzoxazin-3-yl)-acetylamino]-benzoyl}-ampicillin Natriumsalz (Formel I mit R¹ = COOCH₃, R² = H, R³ = R¹⁸ mit R¹⁰, R¹¹ = H, R¹² = R¹⁹ in 3-Position, COR⁹ in 5-Position, R⁹ = N-Ampicillino (Na-Salz), R15, R14 = H, n = 1, R¹, R² wie oben)
(a) Herstellung von N-{3,5-Bis-[(8-methoxycarbonyloxy-2,4-dioxo-1,3-benzoxazin-3-yl)-acetylamino]-benzoyl}-ampicillin
   Die Verbindung wurde analog zu Substanz 12 aus Substanz 27 und Ampicillin-Natriumsalz hergestellt. Erhalten wurde ein weißes Pulver in einer Ausbeute von 90% der Theorie.
(b) Natriumsalz: Das Salz wurde analog zu Substanz 18 aus N-{3,5-Bis-[8-methoxycarbonyloxy-2,4-dioxo-1,3-benzoxazin-3-yl)-acetylamino]-benzoyl}-ampicillin und Natrium-2-ethylhexanoat in Essigsäureethylester hergestellt. Erhalten wurde ein weißes Pulver in einer Ausbeute von 17% der Theorie.

### Beispiel 29

### Herstellung von 4-Chlor-3-(8-methoxycarbonyloxy-2,4-dioxo-1,3-benzoxazin-3-yl)-benzoesäure (Formel I mit R¹ = COOCH₃, R² = H, R³ = R²⁰ mit R¹³ = Cl in 2-Position, COR⁹ in 5-Position, R⁹ = OH, p = 0)

Die Verbindung wurde analog zu Substanz 5 aus 3-Amino-4-chlor-benzoesäure und 2,3-Di-(methoxycarbonyloxy)-benzoylchlorid in wäßriger Natriumhydrogencarbonatlösung hergestellt. Nach Umkristallisation aus Essigsäureethylester wurden farblose Kristalle mit einem Schmelzpunkt von 234-236°C in einer Ausbeute von 41% der Theorie erhalten.

### Beispiel 30

Herstellung von N-[4-Chlor-3-(8-methoxycarbonyloxy-2,4-dioxo-1,3-benzoxazin-3-yl)-benzoyl]-ampicillin Natriumsalz (Formel I mit R¹ = COOCH₃, R² = H, R³ = R²⁰ mit R¹³ = Cl in 2-Position, COR⁹ in 5-Position, R⁹= N-Ampicillino (Na-Salz), p = 0)
(a) Herstellung von 4-Chlor-3-(8-methoxycarbonyloxy-2,4-dioxo-1,3-benzoxazin-3-yl)-benzoylchlorid
   Die Verbindung wurde analog zu Substanz 10a aus Substanz 29 und Phosphorpentachlorid in Tetrachlorkohlenstoff hergestellt. Erhalten wurde ein gelbliches Pulver in einer Ausbeute von 94% der Theorie mit einem Schmelzpunkt von 76-78°C.
(b) Herstellung von N-[4-Chlor-3-(8-methoxycarbonyloxy-2,4-dioxo-1,3- 3benzoxazin-3-yl)-benzoyl]-ampicillin
   Die Verbindung wurde analog zu Substanz 10b aus 4-Chlor-3-(8-methoxycarbonyloxy-2,4-dioxo- 1,3-benzoxazin-3-yl )-benzoylchlorid und Ampicillin-Natriumsalz hergestellt. Erhalten wurde ein weißes Pulver in einer Ausbeute von 87% der Theorie.
(c) Natriumsalz: Es wurde analog zu Substanz 18 aus N-[4-Chlor-3-(8-methoxycarbonyloxy-2,4-dioxo-1,3-benzoxazin-3-yl)-benzoyl]-ampicillin und Natrium-2-ethylhexanoat in Essigsäureethylester hergestellt. Erhalten wurde ein weißes Pulver in einer Ausbeute von 55% der Theorie.

### Beispiel 31

Herstellung von 2-Hydroxy-4-(8-methoxycarbonyloxy-2,4-dioxo-1,3-benzoxazin-3-yl)-benzoesäure (Formel I mit R¹ = COOCH₃, R² = H, R³ = R²⁰ mit R¹³ = OH in 3-Position, COR⁹ in 4-Position, R⁹ = OH, p = 0)

Die Verbindung wurde analog zu Substanz 5 aus 4-Aminosalicylsäure und 2,3-Di-(methoxycarbonyloxy)-benzoylchlorid in wäßriger Natriumhydrogencarbonatlösung hergestellt. Nach Umkristallisation aus Essigsäureethylester wurden farblose Kristalle mit einem Schmelzpunkt von 261-262°C in einer Ausbeute von 68% der Theorie erhalten.

### Beispiel 32

Herstellung von N-[2-Hydroxy-4-(8-methoxycarbonyloxy-2,4-dioxo-1,3-benzoxazin-3-yl)-benzoyl]-ampicillin Natriumsalz (Formel I mit R¹ = COOCH₃, R² = H, R³ = R²⁰ mit R¹³ = OH in 3-Position, COR⁹ in 4-Position, R⁹ = N-Ampicillino (Na-Salz), p = 0),
(a) Herstellung von Succinimido-2-hydroxy-4-(8-methoxycarbonyloxy-2,4-dioxo-1,3-benzoxazin-3-yl)-benzoat: Zu einer Lösung von 0,224g Substanz 31 und 0,069 g N-Hydroxysuccinimid in 5 ml wasserfreiem Dioxan wurde unter Argonatmosphäre und Rühren bei 0°C eine Lösung von 0,124 g Dicyclohexylcarbodiimid in 5 ml wasserfreiem Dioxan gegeben. Das Gemisch wurde 8 Stunden bei 20° C gerührt, der entstandene Niederschlag wurde abfiltriert und das Lösungsmittel im Vakuum entfernt. Das zurückbleibende Öl wurde durch Verreiben mit etwas Isopropanol fest. Umkristallisation aus Essigsäureethylester ergab ein weißes Pulver in einer Ausbeute von 0,23g (81% der Theorie) mit einem Schmelzpunkt von 145-150°C.
(b) Herstellung von N-[2-Hydroxy-4-(8-methoxycarbonyloxy-2,4-dioxo-1,3-benzoxazin-3-yl)-benzoyl]-ampicillin: 0,191 g Ampicillintrihydrat wurden unter Argonatmosphäre in einem Gemisch aus 5 ml Tetrahydrofuran und 5 ml Wasser suspendiert und mit 138 µl Triethylamin in Lösung gebracht. Unter Rühren wurde anschließend eine Lösung von 0,223 g Succinimido-2-hydroxy-4-(8-methoxycarbonyloxy-2,4-dioxo-1,3-benzoxazin-3-yl )-benzoat in 5 ml Tetrahydrofuran zugegeben und die Mischung 10 Stunden bei 20° C gerührt. Das Reaktionsgemisch wurde bei 20° C eingedampft und der Rückstand mit Wasser und Essigsäureethylester versetzt. Nach Ansäuern wurde die organische Phase abgetrennt, mit einer gesättigten Natriumchloridlösung und mit Wasser gewaschen, über Natriumsulfat getrocknet und schließlich weitgehend eingedampft. Durch Zusatz von Petrolether wurde das Ampicillinderivat ausgefällt, das in einer Ausbeute von 0,28 g (84% der Theorie) erhalten wurde.
(c) Natriumsalz: Das Salz wurde analog zu Substanz 18 aus N-[2-Hydroxy-4-(8-methoxycarbonyloxy-2,4-dioxo-1,3-benzoxazin-3-yl)-benzoyl]-ampicillin undNatrium-2-ethylhexanoat in Essigsäureethylester hergestellt. Erhalten wurde ein weißes Pulver in einer Ausbeute von 19% der Theorie.

### Beispiel 33

Herstellung von 3-(8-Methoxycarbonyloxy-2,4-dioxo-1,3-benzoxazin-3-yl)-benzoesäure (Formel I mit R¹ = COOCH₃, R² = H, R³ = R²⁰ mit R¹³ = H , COR⁹ in 3-Position, R⁹ = OH, p = 0)

Die Verbindung wurde analog zu Substanz 5 aus 3-Aminobenzoesäure und 2,3-Di-(methoxycarbonyloxy)-benzoylchlorid in wäßriger Natriumhydrogencarbonatlösung hergestellt. Nach Umkristallisation aus Essigsäureethylester wurden farblose Kristalle in einer Ausbeute von 71% der Theorie mit einem Schmelzpunkt von 250-253°C erhalten.

### Beispiel 34

Herstellung von N-[3-(8-Methoxycarbonyloxy-2,4-dioxo-1,3-benzoxazin-3-yl)-benzoyl]-ampicillin Natriumsalz (Formel I mit R¹ = COOCH₃, R² = H, R³ = R²⁰ mit R¹³ = H, COR⁹ in 3-Position, R⁹ = N-Ampicillino (Na-Salz), p = 0)
(a) Herstellung von 3-(8-Methoxycarbonyloxy-2,4-dioxo-1,3-benzoxazin-3-yl)-benzoylchlorid
   Die Verbindung wurde analog zu Substanz 10a aus Substanz 33 und Phosphorpentachlorid in Tetrachlorkohlenstoff hergestellt. Erhalten wurde ein gelbliches Öl in einer Ausbeute von 97% der Theorie.
(b) Herstellung von N-[3-(8-Methoxycarbonyloxy-2,4-dioxo-1,3-benzoxazin-3-yl)-benzoyl]-ampicillin
   Die Verbindung wurde analog zu Substanz 10b aus 3-(8-Methoxycarbonyl-oxy-2,4-dioxo-1,3-benzoxazin-3-yl)-benzoylchlorid und Ampicillin-Natriumsalz hergestellt. Erhalten wurde ein weißes Pulver in einer Ausbeute von 87% der Theorie.
(c) Natriumsalz: Das Salz wurde analog zu Substanz 18 aus N-[3-(8-Methoxycarbonyloxy-2,4-dioxo-1,3-benzoxazin-3-yl)-benzoyl]-ampicillin und Natrium-2-ethylhexanoat in Essigsäureethylester hergestellt. Erhalten wurde ein weißes Pulver in einer Ausbeute von 59% der Theorie.

### Beispiel 35

Herstellung von 2L-(8-Methoxycarbonyloxy-2,4-dioxo-1,3-benzoxazin-3-yl)-pentandicarbonsäure-1-benzylester (Formel I mit R¹ = COOCH₃, R² = H, R³ = CHR⁴-COR⁵ mit R⁴ = (CH₂)₂COOH, R⁵ = O-Benzyl)

1g L-Glutaminsäure-1-benzylester wurden unter einer Argonatmosphäre in 40 ml wasserfreiem Tetrahydrofuran gelöst. Unter Eiskühlung und Rühren wurden zunächst 1,24 ml Triethylamin, dann eine Lösung von 1,22 g 2,3-Di-(methoxycarbonyloxy)-benzoylchlorid in 10 ml wasserfreiem Tetrahydrofuran hinzugefügt. Nach 20 Stunden Rühren bei 20° C wurde das Tetrahydrofuran im Vakuum entfernt und der Rückstand mit Wasser und Essigsäureethylester versetzt. Unter Eiskühlung und Rühren wurde angesäuert, danach die Essigsäureethylesterphase abgetrennt. Es wurde mehrmals mit Wasser und gesättigter Natriumchloridlösung gewaschen und schließlich eingedampft. Die Reinigung erfolgte mittels präparativer HPLC. Erhalten wurde ein gelblicher Schaum in einer Ausbeute von 0,260 g (13% der Theorie).

### Beispiel 36

Herstellung von 4-Ampicillinocarbamoyl-2L-(8-methoxycarbonyloxy-2,4-dioxo-1,3-benzoxazin-3-yl)-buttersäurebenzylester Natriumsalz (Formel I mit R¹ = COOCH₃, R² = H, R³ = CHR⁴-COR⁵ mit R⁴ = (CH₂)₂CO-N-Ampicillino (Na-Salz), R⁵ = O-Benzyl)
(a) Herstellung von 4-Chlorcarbonyl-2L-(8-methoxycarbonyloxy-2,4-dioxo-1,3-benzoxazin-3-yl)-buttersäurebenzylester
   Die Verbindung wurde analog zu Substanz 10a aus Substanz 35 und Phosphorpentachlorid in Tetrachlorkohlenstoff hergestellt. Erhalten wurde ein gelbliches Öl in einer Ausbeute von 97% der Theorie.
(b) Herstellung von 4-Ampicillinocarbamoyl-2L-(8-methoxycarbonyloxy-2,4-dioxo-1,3-benzoxazin-3-yl)-buttersäurebenzylester
   Die Verbindung wurde analog zu Substanz 10b aus 4-Chlorcarbonyl-2L-(8-methoxycarbonyloxy-2,4-dioxo-1,3-benzoxazin-3-yl)-buttersäurebenzylester und Ampicillin-Natriumsalz hergestellt. Erhalten wurde ein weißes Pulver in einer Ausbeute von 87% der Theorie.
(c) Natriumsalz: Es wurde analog zu Substanz 18 sind aus 4-Ampicillinocarbamoyl-2L-(8-methoxy-carbonyloxy-2,4-dioxo-1,3-benzoxazin-3-yl)-butanoyl]-ampicillin und Natrium-2-ethylhexanoat in Essigsäureethylester hergestellt. Erhalten wurde ein weißes Pulver in einer Ausbeute von 46% der Theorie.

### Beispiel 37

Herstellung von 2L-[2L,6-Bis-(8-methoxycarbonyloxy-2,4-dioxo-1,3-benzoxazin-3-yl)-hexanoylamino]-6-(8-methoxycarbonyloxy-2,4-dioxo-1,3-benzoxazin-3-yl)-hexansäure (Formel I mit R¹ = COOCH₃, R² = H, R³ = CHR⁴-COR⁵ mit R⁴ =(CH₂)₄-Y, R⁵ =R¹⁷ mit X = OH, n = 4)
(a) Herstellung von 2L-[2L,6-Bis-(8-methoxycarbonyloxy-2,4-dioxo-1,3-benzoxazin-3-yl)-hexanoylamino]-6-(8-methoxycarbonyloxy-2,4-dioxo-1,3-benzoxazin-3-yl)-hexansäurebenzylester: 1,55 g 6-Amino-2-(2,6-diaminohexanoylamino)-hexansäurebenzylester-tris-p-toluolsulfonat wurden unter Argonatmosphäre in 20 ml wasserfreiem Dimethylformamid gelöst und die Lösung unter Eiskühlung und Rühren zunächst mit 1,48 ml Triethylamin und dann mit einer Lösung von 1,53 g 2,3-Di-(methoxycarbonyloxy)-benzoylchlorid in 10 ml wasserfreiem Dimethylformamid versetzt. Nach 5 Stunden Rühren bei 0°C und 20 Stunden bei 20° C wurde das Dimethylformamid im Vakuum entfernt und der Rückstand mit Wasser und Essigsäureethylester versetzt. Unter Eiskühlung und Rühren wurde angesäuert und danach die Essigsäureethylesterphase abgetrennt. Es wurde mehrmals mit Wasser und gesättigter Natriumchloridlösung gewaschen und schließlich eingedampft. Die Reinigung erfolgte mittels präparativer HPLC (Elutionsmittel: Acetonitril/Wasser = 1/1 mit 0,5 % Trifluoressigsäure), wobei 0,25 g eines gelblichen Öls (14% der Theorie) zurückblieben.
(b) 0,250 g 2L-[2L,6-Bis-(8-methoxycarbonyloxy-2,4-dioxo-1,3-benzoxazin-3-yl)-hexanoylamino]-6-(8-methoxycarbonyloxy-2,4-dioxo-1,3-benzoxazin-3-yl)-hexansäurebenzylester wurden in 30 ml Ethanol über 0,06 g Palladium auf Aktivkohle (10% Pd) bei 20° C und Normaldruck katalytisch hydriert. Nach Filtration über Celite wurde eingedampft und aus Essigsäureethylester verfestigt. Erhalten wurde ein gelblicher Schaum in einer Ausbeute von 0,220 g (98% der Theorie).

### Beispiel 38

Herstellung von N-{2L-[2L,6-Bis-(8-methoxycarbonyloxy-2,4-dioxo-1,3-benzoxazin-3-yl)-hexanoylamino]-6-(8-methoxycarbonyloxy-2,4-dioxo-1,3-benzoxazin-3-yl)- hexanoyl}-ampicillin Natriumsalz (Formel I mit R¹ = COOCH₃, R² = H, R³ = CHR⁴-COR⁵ mit R⁴ = (CH₂)₄-Y, R⁵ =R¹⁷ mit X = N-Ampicillino (Na-Salz), n = 4)
(a) Herstellung von N-{2L-[2'L,6'-Bis-(8-methoxycarbonyloxy-2,4-dioxo-1,3-benzoxazin-3-yl )-hexanoylamino]-6-(8-methoxycarbonyloxy-2,4-dioxo-1,3-benzoxazin-3-yl)-hexanoyl}-ampicillin
   Die Verbindung wurde analog zu Substanz 12 aus Substanz 37 und Ampicillin-Natriumsalz hergestellt. Erhalten wurde ein weißes Pulver in einer Ausbeute von 90% der Theorie.
(b) Natriumsalz: Das Salz wurde analog zu Substanz 18 aus N-{2L-[2L,6-Bis-(8-methoxycarbonyloxy-2,4-dioxo-1,3-benzoxazin-3-yl)-hexanoylamino]-6-(8-methoxycarbonyloxy-2,4-dioxo-1,3-benz-oxazin-3-yl)-hexanoyl}-ampicillin und Natrium-2-ethylhexanoat in Essigsäureethylester hergestellt. Erhalten wurde ein weißes Pulver in einer Ausbeute von 15% der Theorie.

### Beispiel 39

### Herstellung von 3-Hydroxy-2L-(8-methoxycarbonyloxy-2,4-dioxo-1,3-benzoxazin-3-yl)-propansäure (Formel I mit R¹ = COOCH₃, R² = H, R³ = CHR⁴-COR⁵ mit R⁴ = CH₂-OH, R⁵ = OH)

(a) Herstellung von 2L-N-(2,3-Dimethoxycarbonyloxybenzoyl)-serinbenzylester
   Die Verbindung wurde analog zu Substanz 35 aus L-Serinbenzylesterhydrochlorid und 2,3-Di-(methoxycarbonyloxy)-benzoylchlorid in Tetrahydrofuran hergestellt. Erhalten wurde weißer Schaum in einer Ausbeute von 43% der Theorie.
(b) 1,3 g 2L-N-(2,3-Dimethoxycarbonyloxybenzoyl)-serinbenzylester wurden in 50 ml Ethanol suspendiert und 300 mg Palladium-Kohle-Katalysator (10%ig) hinzugefügt. Nach Überführung in eine Wasserstoffatmosphäre wurde das Gemisch 2 Stunden bei 20° C und Normaldruck geschüttelt. Nach Filtration über Celite wurde eingedampft. Reinigung mittels präparativer HPLC ergab weißen Schaum, Ausbeute: 599 mg (48% der Theorie).

### Beispiel 40

Herstellung von N-[3-Hydroxy-2L-(8-methoxycarbonyloxy-2,4-dioxo-1,3-benzoxazin-3-yl)-propionyl]-ampicillin Natriumsalz (Formel I mit R¹ = COOCH₃, R² = H, R³=CHR⁴-COR⁵ mit R⁴ CH₂-OH, R⁵ N-Ampicillino (Na-Salz)
(a) Herstellung von N-[3-Hydroxy-2L-(8-methoxycarbonyloxy-2,4-dioxo-1,3-benzoxazin-3-yl)-propionyl]-ampicillin
   Die Verbindung wurde analog zu Substanz 12 aus Substanz 39 und Ampicillin-Natriumsalz hergestellt. Erhalten wurde ein weißes Pulver mit einer Ausbeute von 90% der Theorie.
(b) Natriumsalz: Es wurde analog zu Substanz 18 aus N-[3-Hydroxy-2L-(8-methoxycarbonyloxy-2,4-dioxo-1,3-benzoxazin-3-yl)-propionyl]-ampicillin und Natrium-2-ethylhexanoat in Essigsäureethylester hergestellt. Erhalten wurde ein weißes Pulver in einer Ausbeute von 10% der Theorie.

### Beispiel 41

Herstellung von (8-Ethoxycarbonyloxy-2,4-dioxo-1,3-benzoxazin-3-yl)-essigsäure (Formel I mit R¹ = COOC₂H₅, R² = H, R³ = CH₂COOH)

Die Verbindung wurde analog zu Substanz 1 aus Glycin und 2,3-Di-(ethoxycarbonyloxy)-benzoylchlorid in wäßriger Natriumhydrogencarbonatlösung hergestellt. Nach Umkristallisation aus Essigsäureethylester wurden farblose Kristalle mit einem Schmelzpunkt von 162-163°C in einer Ausbeute von 69% der Theorie erhalten.

### Beispiel 42

Herstellung von N-{N'-[6-(8-Methoxycarbonyloxy-2,4-dioxo-1,3-benzoxazin-3-yl)-hexyl]-N'-[2,3-di-(methoxycarbonyloxy)-benzoyl]-6-aminohexyl}-N-[2,3-di-(methoxycarbonyloxy)-benzoyl]-glycin (Formel I mit R³ = Z-CHR⁴-COR⁵; R¹, R7 = COOCH₃,
R², R⁴, R⁶ = H, R⁵ = OH, n = 6, m = 2)
(a) Herstellung des Benzylesters (R5 = OCH₂C₆H₅):
   Zu einer Lösung von 880 mg N-[N'-(6-Aminohexyl)-6-aminohexyl]-glycinbenzylester-tosylat und 1,04 ml Triethylamin in 20 ml Dichlormethan wurde bei -30° C eine Lösung von 864 mg 2,3-Di-(methoxycarbonyloxy)-benzoylchlorid in 5 ml Dichlormethan zugegeben. Die Mischung wurde 1 Stunde bei -10° C und 1 Stunde bei Raumtemperatur gerührt und dann filtriert. Die Lösung wurde eingeengt und der Rückstand in 20 ml Essigsäureethylester aufgenommen. Die Essigsäureethylesterlösung wurde je dreimal mit 1M Salzsäure, gesättigter Natriumbicarbonatlösung und mit Wasser gewaschen. Nach Trocknen und Abdestillieren des Lösungsmittels im Vakuum wurde ein farbloser Feststoff mit einer Ausbeute von 650 mg (60 % der Theorie) erhalten.
(b) Herstellung der Säure (R⁵ = OH):
   Der obige Benzylester wurde in Methanol mit 100 mg Pd/C (10%ig) 3 Stunden lang in einer Schüttelapparatur bei Raumtemperatur hydriert.

Nach Abfiltrieren des Katalysators über Celite wurde das Lösungsmittel im Vakuum abdestilliert. Es wurde ein farbloser Feststoff mit einer Ausbeute von 70 % der Theorie erhalten.

**Tabelle 1**

| **Wachstumszonen (in mm) von Siderophor-Indikatorstämmen mit ausgewählten Benzoxazindionderivaten** | | | | | |
|---|---|---|---|---|---|
| **Substanz- Nr.** | ***Pseud. aeruginosa*** | ***Y.enterocol.*** | ***E.coli*** | ***Salmonella*** | |
| | PAO 6609 | WAH 5030 | AB 2847 | enb-7 | TA 2700 |
| 1 | 0 | | 15 | 18 | 13 |
| 2 | 13 | | 10 | 22 | 21 |
| 4 | 0 | | 0 | 14 | 0 |
| 7 | 0 | | 0 | 10 | 0 |
| 9 | 0 | 20 | 24 | 0 | 16 |
| Kontrolle | a) 35 | a) 40 | b) 23 | c) 38 | c) 20 |

| | | | | | |
|---|---|---|---|---|---|
| a) Ferrioxamin E, | | | | | |
| b) Ferrichrom, | | | | | |
| c) Ferrioxamin G | | | | | |

**Tabelle 2**

| **Antibakterielle Aktivität von Benzoxazindion-Antibiotika-Konjugaten** MHK-Werte mg/ml | | | | | | | |
|---|---|---|---|---|---|---|---|
| Substanz | Pseudomonas aeruginosa | | | Klebsiella | E.coli | Stenotrop. maltoph. | Staphylococcus |
| | SG 137 | ATCC 27853 | NCTC 10662 | ATCC 10031 | ATCC 25922 | GN 12873 | SG 511 |
| Azlocillin | 3,12 | 3,12 | 6,25 | 6,25 | 6,25 | 12,5 | 0,2 |
| Ampicillin | >100 | >100 | >100 | 25 | 6,25 | >100 | <0,05 |
| Substanz 10 | 0,78 | 6,25 | 3,12 | 0,2 | 6,25 | 3,12 | 0,78 |
| Substanz 11 | 0,78 | 3,12 | 3,12 | | 12,5 | 12,5 | 0,78 |
| Substanz 12 | 0,4 | 3,12 | 1,56 | 6,25 | 3,12 | 1,56 | 0,78 |
| Substanz 13 | 0,2 | 1,56 | 3,12 | 0,1 | 3,12 | 3,12 | 1,56 |
| Substanz 14 | 1,56 | 3,12 | 1,56 | 0,78 | 3,12 | 1,56 | 1,56 |
| Substanz 15 | 0,78 | 1,56 | 1,56 | 3,12 | 12,5 | 6,25 | 1,56 |
| Substanz 18 | 0,4 | 1,56 | 1,56 | 6,25 | 25 | 3,12 | 1,56 |
| Substanz 20 | 1,56 | 6,25 | 12,5 | 6,25 | 12,5 | 6,25 | 0,78 |
| Substanz 22 | 0,2 | 1,56 | 0,78 | 6,25 | 25 | 0,78 | 25 |
| Substanz 24 | 3,12 | 6,25 | 12,5 | 50 | 25 | 25 | 12,5 |
| Substanz 26 | 0,05 | 0,2 | 0,1 | 0,2 | 6,25 | 1,56 | 6,25 |
| Substanz 28 | 0,05 | 1,56 | 1,56 | 1,56 | 25 | 3,12 | 25 |
| Substanz 30 | <0,05 | 0,78 | 0,4 | 1,56 | 6,25 | 0,78 | 1,56 |
| Substanz 32 | 0,1 | 0,78 | 0,78 | 3,12 | 50 | 0,78 | 0,4 |
| Substanz 34 | <0,05 | 0,2 | 0,4 | 1,56 | 3,12 | 1,56 | 0,78 |
| Substanz 36 | 0,2 | 0,78 | 1,56 | 1,56 | 3,12 | 1,56 | 0,2 |
| Substanz 38 | 0,2 | 0,78 | 0,78 | 0,4 | 6,25 | 0,4 | 3,12 |
| Substanz 40 | 0,4 | 1,56 | 1,56 | 25 | 12,5 | 3,12 | 0,4 |

In den Beispielen bedeutet HPLC High Performance Liquid Chromatography

## Patentansprüche

1. Benzoxazindionderivate der Formel I 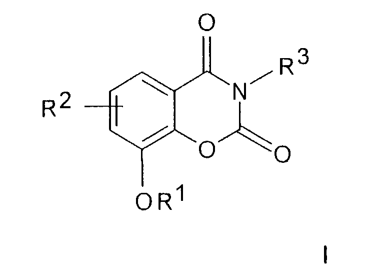 in welcher R¹ = H, COAlkyl oder COOAlkyl, wobei Alkyl jeweils geradkettiges oder verzweigtes C₁₋₈-Alkyl bedeutet. R² = H, geradkettiges oder verzweigtes C₁₋₈-Alkyl, Halogen und R³ folgende Substituenten darstellen:
a) R³ = -Z-CHR⁴-COR⁵
mit Z = 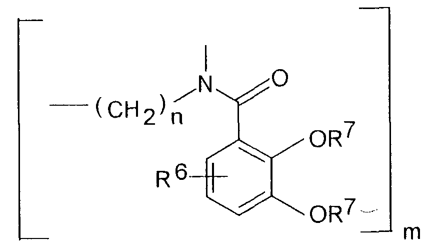 mit R⁴ = H, geradkettiges oder verzweigtes C₁₋₈-Alkyl, Phenyl oder substituiertes Phenyl, das durch geradkettiges oder verzweigtes C₁₋ ₈-Alkyl, Halogen, insbesondere Cl oder F, geradkettiges oder verzweigtes C₁₋₈-Alkoxy, Hydroxy, Carboxy, geradkettiges oder verzweigtes C₁₋₈-Alkoxycarbonyl oder halogensubstituiertes C₁₋₈-Alkyl substituiert ist, insbesondere Hydroxy-oder Acyloxyphenyl, wobei Acyl geradkettiges oder verzweigtes C₁₋₆-Alkanoyl oder geradkettiges oder verzweigtes C₁₋₆-Alkoxycarbonyl bedeutet,
oder mit R⁴ = (CH₂)ₙCOX mit X = OA, wobei A = H, geradkettiges oder verzweigtes C₁₋₈-Alkyl, ein Natrium- oder Kaliumion oder ein Ammoniumion bzw. ein ein- bis vierfach alkylsubstituiertes Ammoniumion ist, oder mit X = ein Wirkstoffrest, der den Rest eines Antibiotikums , welches über eine freie OH- oder NH-Gruppe direkt oder über gebräuchliche Linkergruppen mit dem restlichen Strukturteil der Verbindung der Formel I verbunden ist, ausgewählt aus der β-Lactam-Antibiotika, wobei diese insbesondere Penicilline, Cephalosporine oder Carbapeneme sind, Tetracycline, Aminoglykoside, Makrolide oder Chinolone umfassenden Gruppe bedeutet, und mit n = 1 - 10,
oder mit R⁴ = (CH₂)ₙ-Y, wobei Y einen Benzoxazindionrest der Form 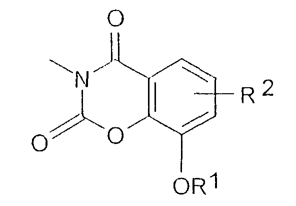 darstellt, wobei R¹ und R² wie oben definiert sind, und beide Benzoxazindionreste gleich oder verschieden sein können, und n = 1-10 sein kann,
und mit R⁵ = OA, wobei A wie oben definiert ist oder Benzyl bedeutet,
oder mit R⁵ = ein Wirkstoffrest wie oben definiert
oder mit R⁵ = NH-CHR⁸-COR⁹, mit R⁸ = H, geradkettiges oder verzweigtes C₁₋₈-Alkyl, Phenyl oder substituiertes Phenyl wie oben definiert und mit R⁹ = OA, wobei A wie oben definiert ist, oder mit R⁹ = ein Wirkstoffrest wie oben definiert
oder mit R⁵= 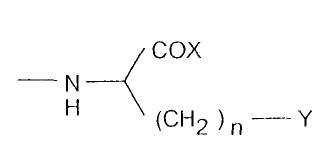 = R¹⁷ mit X und Y wie oben, n = 1 -10 und mit R⁶ = H, geradkettiges oder verzweigtes C₁₋₈-Alkyl, Halogen, und mit R⁷ = H, Acyl wie oben definiert und n = 1 - 10 und m = 1 - 2, mit der Maßgabe, daß wenn R⁴ keinen Wirkstoffrest wie oben definiert enthält, R⁵ oder R⁹ einen Wirkstoffrest wie oben definiert darstellen oder enthalten, oder wenn R⁵ keinen Wirkstoffrest wie oben definiert enthält, R⁴ = (CH₂)ₙ COX mit X = ein Wirkstoffrest wie oben definiert bedeutet, oder
b) R³ = CHR⁴-COR⁵ mit R⁴ und R⁵ wie oben oder R⁴ = CH₂OH mit der Maßgabe, daß wenn R⁴ keinen Wirkstoffrest wie oben definiert enthält, R⁵ oder R⁹ einen Wirkstoffrest wie oben definiert darstellen, oder wenn R⁵ keinen Wirkstoffrest wie oben definiert enthält, R⁴ = (CH₂)ₙ COX mit X = ein Wirkstoffrest wie oben definiert bedeutet, oder
c) R³ = 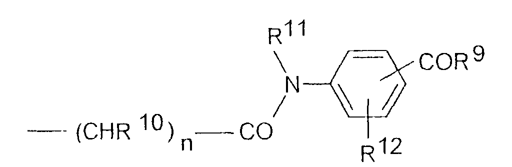 = R¹⁸ mit R¹⁰ und/oder R¹¹ = H, geradkettiges oder verzweigtes C₁₋₈- Alkyl, Phenyl oder substituiertes Phenyl wie oben definiert, n = 1 -10, und mit COR⁹ und R¹² in allen möglichen Positionen, R⁹ = ein Wirkstoffrest wie oben definiert und R¹² = H, geradkettiges oder verzweigtes C₁₋₈- Alkyl, Halogen, Hydroxy, geradkettiges oder verzweigtes C₁₋₈-Alkoxy, ein Benzoxazindionrest Y oder R¹² = 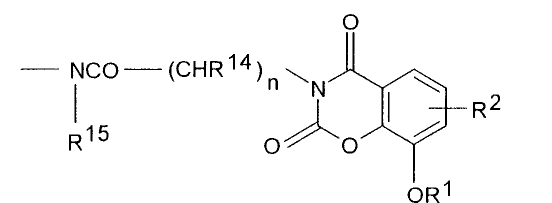 = R¹⁹
mit R¹, R² wie oben, R¹⁴, R¹⁵ wie R¹, R² und n = 1 - 10 sein kann, oder
d) R³ = 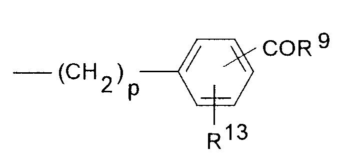 = R²⁰ mit R¹³ und COR⁹ in allen möglichen Positionen und mit R¹³ = H, geradkettiges oder verzweigtes C₁₋₈-Alkyl, Halogen, Hydroxy, geradkettiges oder verzweigtes C₁₋₈-Alkoxy oder ein Benzoxazindionrest Y, und
R⁹ = ein Wirkstoffrest wie oben definiert und p= 0 - 2, oder
e) R³ = 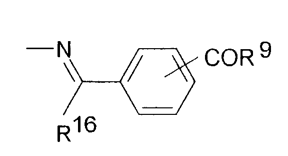 = R²¹ oder R³ = 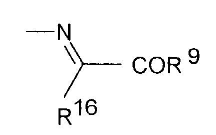 = R²²
mit R⁹ = ein Wirkstoffrest wie oben definiert, R¹⁶ = H, geradkettiges oder verzweigtes C₁₋₈-Alkyl, Phenyl oder substituiertes Phenyl wie oben definiert, oder
f) R³ = ein Wirkstoffrest wie oben definiert
sowie unter physiologischen Bedingungen spaltbare Ester von solchen Verbindungen der Formel I, die eine freie Carboxylgruppe im Rest R³ aufweisen.

2. Verbindungen der Formel I gemäß Anspruch 1, wobei X oder R³ oder R⁵ oder R⁹ der Rest eines antibakteriellen Wirkstoffes wie oben definiert bedeutet.

3. Verbindungen der Formel I gemäß Anspruch 1, wobei X oder R³ oder R⁵ oder R⁹ der Rest eines beliebigen Cephalosporins ist, welcher über eine NH- oder eine OH-Gruppe gebunden ist.

4. Verbindungen der Formel I gemäß Anspruch 1, wobei X oder R³ oder R⁵ oder R⁹ der Rest eines beliebigen Penicillins ist, welches über eine NH- oder eine OH-Gruppe gebunden ist.

5. Verbindungen der Formel I gemäß Anspruch 1 oder 4, wobei X oder R³ oder R⁵ oder R⁹ ein Ampicillinrest bedeutet.

6. Verbindungen der Formel 1 gemäß Anspruch 1 oder 4, wobei X oder R³ oder R⁵ oder R⁹ ein Amoxicillin- oder O-Acylamoxicillinrest bedeutet.

7. Verbindungen der Formel I gemäß Anspruch 1, wobei X oder R³ oder R⁵ oder R⁹ einen 6-Aminopenicillansäurerest bedeutet.

8. Verbindungen der Formel I gemäß Anspruch 1, wobei X oder R³ oder R⁵ oder R⁹ ein beliebiger Tetracyclinrest ist, welcher über eine NH- oder eine OH-Gruppe gebunden ist.

9. Verbindungen der Formel I gemäß Anspruch 1, wobei X oder R³ oder R⁵ oder R⁹ der Rest eines beliebigen Makrolids ist, welches über eine NH- oder eine OH-Gruppe gebunden ist.

10. Verbindungen der Formel I gemäß Anspruch 1, wobei X oder R³ oder R⁵ oder R⁹ der Rest eines beliebigen Chinolons ist, welches über eine NH- oder eine OH-Gruppe gebunden ist.

11. Verbindungen der Formel I gemäß Anspruch 1, wobei X oder R³ oder R⁵ oder R⁹ der Rest eines beliebigen Carbapenems ist, welches über eine NH- oder eine OH-Gruppe gebunden ist.

12. N-(8-Methoxycarbonyloxy-2,4-dioxo-benzoxazin-3-yl-acetyl)-ampicillin gemäß Anspruch 1, 4 oder 5.

13. N-(8-Methoxycarbonyloxy-2,4-dioxo-benzoxazin-3-yl-acetyl )-amoxicillin gemäß Anspruch 1, 4 oder 6.

14. Verbindungen der Formel I nach einem der An-sprüche 1 bis 13 zur Verwendung als Wachstumsfaktoren für Bakterien.

15. Verbindungen der Formel I nach einem der An-sprüche 1 bis 13, enthaltend einen Wirkstoffrest wie oben definiert für X oder R³ oder R⁵ oder R⁹, zur Verwendung als Therapeutika bei bakteriellen Infektionen.

16. Arzneimittel enthaltend eine Verbindung der Formel I nach einem der Ansprüche 1 bis 13 zusammen mit üblichen Trägermaterialien.

17. Verbindungen der Formel I nach einem der Ansprüche 1 bis 13 zur Verwendung als Medikament.

18. Verwendung von Verbindungen der Formel I nach Anspruch 1 mit R⁵ oder R⁹ = OH zur Herstellung von Verbindungen der Formel l nach einem der Ansprüche 1 bis 13.

## Claims

1. Benzoxazinedione derivatives of the formula I 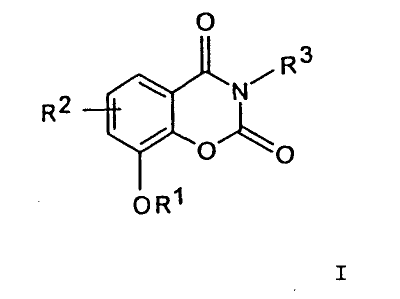 in which R¹ = H, COalkyl or COOalkyl, wherein alkyl in each case denotes straight-chain or branched C₁₋₈-alkyl, R² = H, straight-chain or branched C₁₋₈-alkyl or halogen and R³ represents the following substituents:
a) R³ = -Z-CHR⁴-COR⁵ where Z = 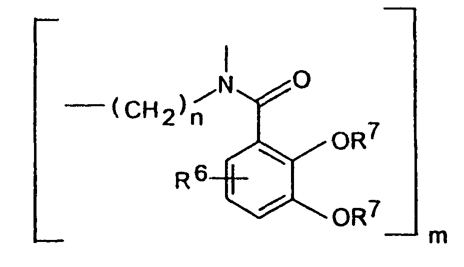 where R⁴ = H, straight-chain or branched C₁₋₈-alkyl, phenyl or substituted phenyl, which is substituted by straight-chain or branched C₁₋₈-alkyl, halogen, in particular Cl or F, straight-chain or branched C₁₋₈-alkoxy, hydroxyl, carboxyl, straight-chain or branched C₁₋₈-alkoxycarbonyl or halogen-substituted C₁₋₈-alkyl, in particular hydroxy- or acyloxyphenyl, wherein acyl denotes straight-chain or branched C₁₋₆-alkanoyl or straight-chain or branched C₁₋₆-alkoxycarbonyl, or where R⁴ = (CH₂)_{N}COX, where X = OA, wherein A = H, straight-chain or branched C₁₋₈-alkyl, a sodium or potassium ion or an ammonium ion or an ammonium ion with one to four alkyl substituents, or where X = an active compound radical which denotes the radical of an antibiotic, which is bonded via a free OH or NH group directly or via conventional linker groups to the remaining structural part of the compound of the formula I, chosen from the group comprising β-lactam antibiotics, these being in particular penicillins, cephalosporins or carbapenems, tetracyclines, aminoglycosides, macrolides or quinolones, and where n = 1 - 10,
or where R⁴ = (CH₂)ₙ-Y, wherein Y represents a benzoxazinedione radical of the form 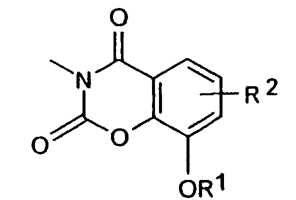 wherein R¹ and R² are as defined above, and the two benzoxazinedione radicals can be identical or different, and n can be 1 - 10,
and where R⁵ = OA, wherein A is as defined above, or denotes benzyl
or where R⁵ = an active compound radical as defined above
or where R⁵ = NH-CHR⁸-COR⁹, where R⁸ = H, straightchain or branched C₁₋₈-alkyl, phenyl or substituted phenyl as defined above and where R⁹ = OA, wherein A is as defined above, or where R⁹ = an active compound radical as defined above or where R⁵ = 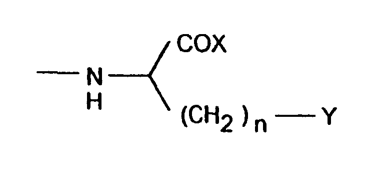 = R¹⁷ where X and Y are as above, n = 1 - 10
and where R⁶ = H, straight-chain or branched C₁₋₈-alkyl or halogen,
and where R⁷ = H or acyl as defined above and n = 1 - 10 and m = 1 - 2, with the proviso that if R⁴ contains no active compound radical as defined above, R⁵ or R⁹ represent or contain an active compound radical as defined above, or if R⁵ contains no active compound radical as defined above, R⁴ = (CH₂)ₙCOX, where X = an active compound radical as defined above, or
b) R³ = CHR⁴-COR⁵ where R⁴ and R⁵ are as above or R⁴ = CH₂OH, with the proviso that if R⁴ contains no active compound radical as defined above, R⁵ or R⁹ represent an active compound radical as defined above, or if R⁵ contains no active compound radical as defined above, R⁴ = (CH₂)ₙCOX, where X = an active compound radical as defined above, or
c) R³ = 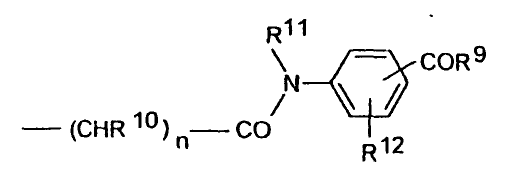 = R¹⁸
where R¹⁰ and/or R¹¹ = H, straight-chain or branched C₁₋₈-alkyl, phenyl or substituted phenyl as defined above, n = 1 - 10, and where COR⁹ and R¹² are in all the possible positions, R⁹ = an active compound radical as defined above
and R¹² = H, straight-chain or branched C₁₋₈-alkyl, halogen, hydroxyl, straight-chain or branched C₁₋₈-alkoxy or a benzoxazinedione radical Y or R¹² = 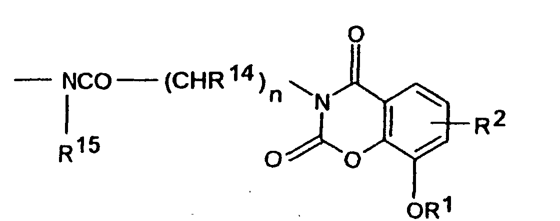 = R¹⁹
where R¹ and R² are as above, R¹⁴ and R¹⁵ are as R¹ and R² and n can be 1 - 10, or
d) R³ = 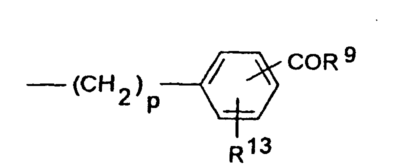 = R²⁰ where R¹³ and COR⁹ are in all the possible positions and where R¹³ = H, straight-chain or branched C₁₋₈-alkyl, halogen, hydroxyl, straight-chain or branched C₁₋₈-alkoxy or a benzoxazinedione radical Y, and
R⁹ = an active compound radical as defined above and p = 0 - 2, or
e) R³ = 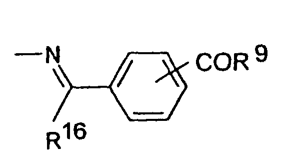 = R²¹ or R³ = 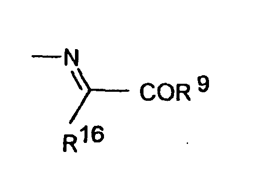 = R²²
where R⁹ = an active compound radical as defined above and R¹⁶ H, straight-chain or branched C₁₋₈-alkyl, phenyl or substituted phenyl as defined above, or
f) R³ = an active compound radical as defined above.
and esters, which can be split off under physiological conditions, of those compounds of the formula I which contain a free carboxyl group in the radical R³.

2. Compounds of the formula I according to claim 1, wherein X or R³ or R⁵ or R⁹ denotes the radical of an antibacterial active compound as defined above.

3. Compounds of the formula I according to claim 1, wherein X or R³ or R⁵ or R⁹ is the radical of any desired cephalosporin, which is bonded via an NH or an OH group.

4. Compounds of the formula I according to claim 1, wherein X or R³ or R⁵ or R⁹ is the radical of any desired penicillin, which is bonded via an NH or an OH group.

5. Compounds of the formula I according to claim 1 or 4, wherein X or R³ or R⁵ or R⁹ denotes an ampicillin radical.

6. Compounds of the formula I according to claim 1 or 4, wherein X or R³ or R⁵ or R⁹ denotes an amoxicillin or O-acylamoxicillin radical.

7. Compounds of the formula I according to claim 1, wherein X or R³ or R⁵ or R⁹ denotes a 6-aminopenicillanic acid radical.

8. Compounds of the formula I according to claim 1, wherein X or R³ or R⁵ or R⁹ is any desired tetracycline radical, which is bonded via an NH or an OH group.

9. Compounds of the formula I according to claim 1, wherein X or R³ or R⁵ or R⁹ is the radical of any desired macrolide, which is bonded via an NH or an OH group.

10. Compounds of the formula I according to claim 1, wherein X or R³ or R⁵ or R⁹ is the radical of any desired quinolone, which is bonded via an NH or an OH group.

11. Compounds of the formula I according to claim 1, wherein X or R³ or R⁵ or R⁹ is the radical of any desired carbapenem, which is bonded via an NH or an OH group.

12. N-(8-Methoxycarbonyloxy-2,4-dioxo-benzoxazin-3-yl-acetyl)-ampicillin according to claim 1, 4 or 5.

13. N-(8-Methoxycarbonyloxy-2,4-dioxo-benzoxazin-3-yl-acetyl)-amoxicillin according to claim 1, 4 or 6.

14. Compounds of the formula I according to one of claims 1 to 13, for use as growth factors for bacteria.

15. Compounds of the formula I according to one of claims 1 to 13 containing an active compound radical as defined above for X or R³ or R⁵ or R⁹, for use as therapeutic agents for bacterial infections.

16. Medicaments comprising a compound of the formula I according to one of claims 1 to 13, together with conventional carrier materials.

17. Compounds of the formula I according to one of claims 1 to 13 for use as a medicament.

18. Use of compounds of the formula I according to claim 1 where R⁵ or R⁹ = OH for the preparation of compounds of the formula I according to one of claims 1 to 13.

## Revendications

1. Dérivés de benzoxazine-dione de formule I 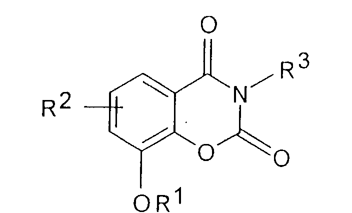 dans laquelle R¹ représente H, un groupe CO-alkyle ou COO-alkyle, dans lequel alkyle désigne dans chaque cas un groupe alkyle en C₁ à C₈ linéaire ou ramifié, R² représente H, un groupe alkyle en C₁ à C₈ linéaire ou ramifié, un halogène, et R³ représente les substituants suivants :
a) R³ = -Z-CHR⁴-COR⁵
avec Z = 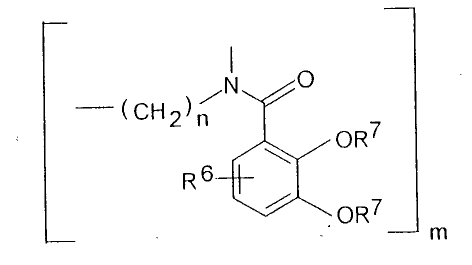 où R⁴ représente H, un groupe alkyle en C₁ à C₈ linéaire ou ramifié, phényle ou phényle substitué, qui est substitué par un radical alkyle en C₁ à C₈ linéaire ou ramifié, un halogène, notamment Cl ou F, un radical alkoxy en C₁ à C₈ linaire ou ramifié, un radical hydroxy, carboxy, un radical (alkoxy en C₁ à C₈)-carbonyle linéaire ou ramifié ou alkyle en C₁ à C₈ halogéné, notamment un groupe hydroxy- ou alcyloxyphényle dont le radical acyle est un radical alcanoyle en C₁ à C₆ linaire ou ramifié ou (alkoxy en C₁ à C₆)-carbonyle linaire ou ramifié,
ou bien R⁴ représente un groupe (CH₂)ₙCOX dans lequel X est un groupe OA où A représente l'hydrogène, un groupe alkyle en C₁ à C₈ linaire ou ramifié, un ion sodium ou potassium ou un ion ammonium ou ammonium portant 1 à 4 substituants alkyle, ou bien X représente un reste de substance active, qui représente le reste d'un antibiotique qui est lié au moyen d'un groupe OH- ou NH libre, directement ou par l'intermédiaire de groupes classiques de jonction, à la partie restante de la structure du composé de formule I, choisi dans le groupe comprenant les antibiotiques du β-lactame, c'est-à-dire en particulier pénicillines, céphalosporine ou carbapénèmes, des tétracyclines, des amino-glycosides, des macrolides ou des chinolones, et n a une valeur de 1 à 10,
ou bien R⁴ représente un groupe (CH₂)ₙ-Y dans lequel Y est un reste de benzoxazine-dione de formule 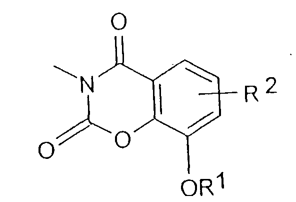 dans laquelle R¹ et R² sont tels que définis ci-dessus et les deux restes de benzoxazine-dione peuvent être identiques ou différents, et n peut avoir une valeur de 1 à 10,
et où R⁵ est un groupe OA dans lequel A est tel que défini ci-dessus ou représente un groupe benzyle,
ou bien R⁵ est un reste de substance active tel que défini ci-dessus,
ou bien R⁵ est un groupe NH-CHR⁸-COR⁹ dans lequel R⁸ représente l'hydrogène, un groupe alkyle en C₁ à C₈ linaire ou ramifié, un groupe phényle ou un groupe phényle substitué tel que défini ci-dessus et R⁹ est un groupe OA dans lequel A est tel que défini ci-dessus, ou bien R⁹ est un reste de substance active tel que défini ci-dessus
ou bien R⁵ est un groupe 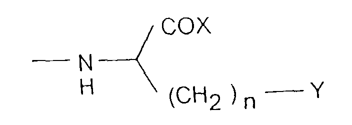 = R¹⁷ dans laquelle X et Y sont tels que définis ci-dessus et n a une valeur de 1 à 10
et où R⁶ représente l'hydrogène, un groupe alkyle en C₁ à C₈ linaire ou ramifié, un halogène, et R⁷ représente l'hydrogène, un groupe acyle tel que défini ci-dessus et
n a une valeur de 1 à 10 et m a la valeur 1 ou 2, sous réserve que, lorsque R⁴ ne contient pas de reste de substance active comme défini ci-dessus, R⁵ ou R⁹ représente ou contient un reste de substance active tel que défini ci-dessus, ou que lorsque R⁵ ne contient pas de reste de substance active comme défini ci-dessus, R⁴ représente un groupe (CH₂)ₙCOX dans lequel X est un reste de substance active tel que défini ci-dessus, ou bien
b) R³ est un groupe CHR⁴-COR⁵
dans lequel R⁴ et R⁵ sont tels que définis ci-dessus ou R⁴ est un groupe CH₂OH sous réserve que, lorsque R⁴ ne contient pas de reste de substance active tel que défini ci-dessus, R⁵ ou R⁹ représente un reste de substance active tel que défini ci-dessus, ou que lorsque R⁵ ne contient pas un reste de substance active comme défini ci-dessus, R⁴ soit un groupe de formule (CH₂)ₙCOX dans laquelle X désigne un reste de substance active tel que défini ci-dessus, ou bien
c) R³ est un groupe de formule générale 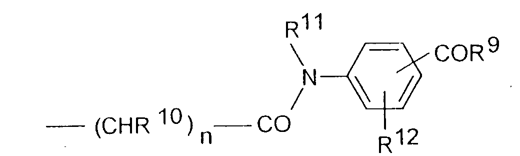 = R¹⁸
dans laquelle R¹⁰ et/ou R¹¹ représentent l'hydrogène, un groupe alkyle en C₁ à C₈ linaire ou ramifié, phényle ou phényle substitué tel que défini ci-dessus, n a une valeur de 1 à 10, et COR⁹ et R¹² occupent toutes positions possibles, R⁹ représente un reste de substance active comme défini ci-dessus et R¹² représente H, un groupe alkyle en C₁ à C₈ linaire ou ramifié, un halogène, un groupe hydroxy, un groupe alkoxy en C₁ à C₈ linéaire ou ramifié, un reste benzoxazine-dione Y ou bien
R¹² est un groupe 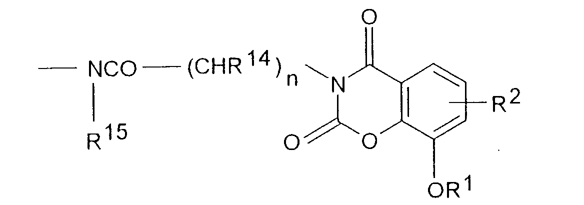 = R¹⁹
dans lequel R¹, R² sont tels que définis ci-dessus, R¹⁴, R¹⁵ sont tels que R¹, R², et n peut avoir une valeur de 1 à 10, ou bien
d) R³ est un groupe 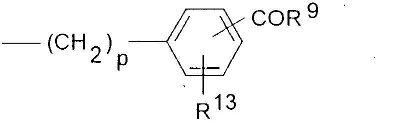 = R²⁰
dans lequel R¹³ et COR⁹ occupent toutes positions possibles et R¹³ représente l'hydrogène, un groupe alkyle en C₁ à C₈ linaire ou ramifié, un halogène, un groupe hydroxy, alkoxy en C₁ à C₈ linaire ou ramifié ou un reste Y de benzoxazine-dione et
R⁹ est un reste de substance active comme défini ci-dessus et p a une valeur de 0 à 2, ou bien
e) R³ représente 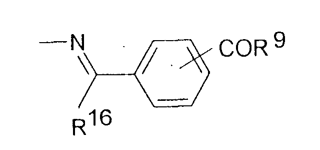 = R²¹ ou R³ représente 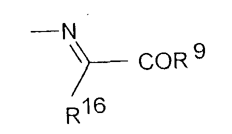 = R²² où R⁹ est un reste de substance active tel que défini ci-dessus, R¹⁶ représente l'hydrogène, un groupe alkyle en C₁ à C₈ linaire ou ramifié, un groupe phényle ou un groupe phényle substitué tel que défini ci-dessus, ou bien
f) R³ est un reste de substance active tel que défini ci-dessus,
de même que les esters, clivables dans les conditions physiologiques, des composés de formule I qui présentent un groupe carboxyle libre dans le reste R³.

2. Composés de formule I suivant la revendication 1, dans laquelle X ou R³ ou R⁵ ou R⁹ désigne le reste d'une substance active antibactérienne telle que définie ci-dessus.

3. Composés de formule I suivant la revendication 1, dans laquelle X ou R³ ou R⁵ ou R⁹ est le reste d'une céphalosporine quelconque qui est liée par un groupe NH- ou par un groupe OH-.

4. Composés de formule I suivant la revendication 1, dans laquelle X ou R³ ou R⁵ ou R⁹ est le reste d'une pénicilline quelconque qui est liée par un groupe NH- ou un groupe OH.

5. Composés de formule I suivant la revendication 1 ou 4, dans laquelle X ou R³ ou R⁵ ou R⁹ désigne un reste d'ampicilline.

6. Composés de formule I suivant la revendication 1 ou 4, dans laquelle X ou R³ ou R⁵ ou R⁹ désigne un reste d'amoxicilline ou de O-acylamoxicilline.

7. Composés de formule I suivant la revendication 1, dans laquelle X ou R³ ou R⁵ ou R⁹ désigne un reste d'acide 6-aminopénicillanique.

8. Composés de formule I suivant la revendication 1, dans laquelle X ou R³ ou R⁵ ou R⁹ est un reste quelconque de tétracycline qui est lié par un groupe NH- ou par un groupe OH-.

9. Composés de formule I suivant la revendication 1, dans laquelle X ou R³ ou R⁵ ou R⁹ est le reste d'un macrolide quelconque qui est lié par un groupe NH- ou par un groupe OH-.

10. Composés de formule I suivant la revendication 1, dans laquelle X ou R³ ou R⁵ ou R⁹ est le reste d'une quinolone quelconque qui est lié par un groupe NH- ou par un groupe OH-.

11. Composés de formule I suivant la revendication 1, dans laquelle X ou R³ ou R⁵ ou R⁹ est le reste d'un carbapénème quelconque qui est lié par un groupe NH- ou par un groupe OH-.

12. N-(8-méthoxycarbonyloxy-2,4-dioxo-benzoxazine-3-yl-acétyl)-ampicilline suivant la revendication 1, 4 ou 5.

13. N-(8-méthoxycarbonyloxy-2,4-dioxo-benzoxazine-3-yl-acétyl)-amoxicilline suivant la revendication 1, 4 ou 6.

14. Composés de formule I suivant l'une des revendications 1 à 13, destinés à être utilisés comme facteurs de croissance pour des bactéries.

15. Composés de formule I suivant l'une quelconque des revendications 1 à 13, contenant un reste de substance active tel que défini ci-dessus pour X ou R³ ou R⁵ ou R⁹, destinés à être utilisés comme agents thérapeutiques dans des infections bactériennes.

16. Médicament contenant un composé de formule I suivant l'une des revendications 1 à 13 en association avec des supports classiques.

17. Composés de formule I suivant l'une des revendications 1 à 13, destinés à être utilisés comme médicaments.

18. Utilisation de composés de formule I suivant la revendication 1 avec R⁵ ou R⁹ = OH pour la préparation de composés de formule I selon l'une des revendications 1 à 13.
